(19) Europäisches Patentamt
European Patent Office
Office européen des brevets

(11) EP 1 391 450 A1

(12) **EUROPEAN PATENT APPLICATION**
published in accordance with Art. 158(3) EPC

(43) Date of publication:
**25.02.2004 Bulletin 2004/09**

(21) Application number: **01930006.0**

(22) Date of filing: **09.05.2001**

(51) Int Cl.[7]: **C07D 207/335**, C07D 213/38, C07D 213/61, C07D 241/12, C07D 231/12, C07D 239/26, C07D 233/64, C07D 277/32, C07D 405/12, C07D 401/12, C07D 413/12, C07D 249/02, A01N 43/40, A01N 43/16, A01N 43/56, A01N 43/54, A01N 43/50, A01N 43/36, A01N 43/78, A01N 43/653

(86) International application number:
**PCT/JP2001/003875**

(87) International publication number:
**WO 2001/085684 (15.11.2001 Gazette 2001/46)**

(84) Designated Contracting States:
**AT BE CH CY DE DK ES FI FR GB GR IE IT LI LU MC NL PT SE TR**

(71) Applicant: **Kureha Chemical Industry Co., Ltd.**
**Tokyo 103-8552 (JP)**

(72) Inventors:
- **ITO, Atsushi**
  **Iwaki-shi, Fukushima 974-8232 (JP)**
- **SUDO, Keiichi**
  **Iwaki-shi, Fukushima 974-8232 (JP)**
- **WATANABE, Tsumoru**
  **Kitaibaraki-shi, Ibaraki 319-1704 (JP)**
- **KUSANO, Nobuyuki**
  **Iwaki-shi, Fukushima 972-8322 (JP)**
- **ARAKI, Nobuyuki**
  **Iwaki-shi, Fukushima 974-8232 (JP)**
- **EIZUKA, Takayoshi**
  **Iwaki-shi, Fukushima 974-8232 (JP)**
- **NIIZEKI, Yoshitaka**
  **Iwaki-shi, Fukushima 974-8232 (JP)**

(74) Representative: **Hayes, Adrian Chetwynd et al**
**Boult Wade Tennant,**
**Verulam Gardens**
**70 Gray's Inn Road**
**London WC1X 8BT (GB)**

(54) **N-(HETEROCYCLE-METHYL)ALKYLAMINE DERIVATIVE, PROCESS FOR PRODUCING THE SAME, AND BACTERICIDE**

(57) A N-heterocyclicmethyl-alkylamine derivative according to the present invention is a N-heterocyclic-methyl-alkylamine derivative represented by the following general formula (I):

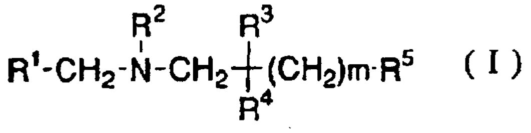

( I )

or an acid addition salt thereof.
[in the formula, $R^1$ represents a heterocycle which has at least one nitrogen atom as a hetero atom and may have a substituent on a ring; $R^2$ represents a hydrogen or the like; $R^3$ represents an alkyl group having a carbon number of 1 to 5 or the like; $R^4$ represents an alkyl group having a carbon number of 1 to 5 or the like; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents a cycloalkyl group or the like described by the following formula (III):

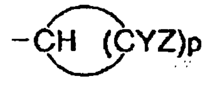

(Ⅲ)

(in the formula, Y and Z each represents a hydrogen

EP 1 391 450 A1

atom or the like, the letter p represents an integer of 2 to 5, and the groups described by CYZ may be each the same or different), when m is 1 and $R^4$ is a hydrogen atom, $R^5$ is a cycloalkyl group describedby the foregoing formula (III).]

## Description

### Technical Field

This invention relates to N-heterocyclicmethyl-alkylamine derivatives, a preparation method thereof and fungicides, and particularly, to novel N-heterocyclicmethyl-alkylamine derivatives which are used for effective ingredients in agricultural and horticultural fungicides, and medicinal antifungal agents, a preparation method thereof, and fungicides containing the same as effective ingredients.

### Background Art

3-phenylpropylamines are commercialized as fungicides such as a compound of N-[3-p-t-butylphenyl-2-methyl-1-propyl]-cis-2,6-dimethy lmorpholine (fenpropimorph) described in Japanese Unexamined Patent PublicationNo. SHO 53-77070 and a compound of N-[3-p-t-butylphenyl-2-methyl-1-propyl] piperidine (fenpropidine) described in Japanese Unexamined Patent Publication No. SHO 53-68785 and Japanese Unexamined Patent Publication No. SHO 53-68786.

Although nitrogen atoms of the amino groups in the foregoing compounds form a part of the ring structures, certain compounds are known that nitrogen atoms of the amino groups do not form as part of the ring structures and a heterocyclicmethyl group is bonded to the nitrogen atom, whose examples are the compounds described in Japanese Unexamined Patent Publication No. SHO 63-258867, wherein the compounds have a heterocyclicmethyl group such as a tetrahydrofurfuryl group and a thenyl group containing oxygen and sulfur, respectively, and the following N-heterocyclicmethylpropylamine derivatives that are described in the literature Pestic. Sci., 35, 339 (1992)., N-[3-(4-t-butylphenyl)-2-methylpropyl]-N-(t-butyl)-3-py ridylmethylamine, N-[3-(4-t-butylphenyl)-2-methylpropyl]-N-butyl-3-pyridy lmethylamine and N-[3-(4-t-butylphenyl)-2-methylpropyl]-N-methyl-3-pyrid ylmethylamine.

Additionally, Publication No. WO99/12902 discloses the N-heterocyclicmethyl-propylamine derivatives, and it is described that the fungicide having the above derivatives as an effective ingredient has a control effect on plant diseases.

However, even the conventional N-heterocyclicmethyl-propylamine derivatives described in the aforementioned Publication No. WO99/12902 are yet insufficient to obtain a fungicide that exhibits a sufficiently high control effect. Thus, the development of a compound which has a more powerful fungicidal activity on various kinds of deleterious organism has been required.

### Disclosure of the Invention

It is an object of the present invention to provide novel N-heterocyclicmethyl-alkylamine derivatives that are fit to use as agricultural and horticultural fungicides and medicinal antifungal agents having a high fungicidal activity on pathogenic fungi in addition to the low toxicity to men and animals and the high safety for handling, and also to provide the preparation method thereof and the fungicides containing them as an effective ingredient.

As a result of assiduous study to accomplish the aforementioned object, the present inventors came to achieve the present invention by finding out that the above issue could be overcome because N-heterocyclicmethyl-alkylamine derivatives having a specific structure had a high fungicidal activity against pathogenic fungi, and could be utilized as an effective ingredient of the fungicides.

In other words, N-heterocyclicmethyl-alkylamine derivatives of the present invention are N-heterocyclicmethyl-alkylamine derivatives described by the following general formula (I) or acid addition salts thereof.

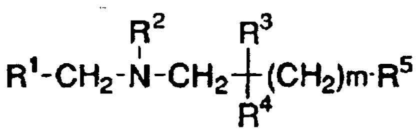

( I )

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring

structure; m represents an integer of 1 to 3; $R^5$ represents one kind selected from a group consisting of a phenyl group and a cycloalkyl group described by the following formulae (II) and (III), respectively. The formula (II) is:

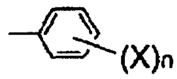

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

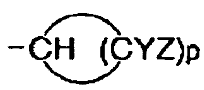

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III).]

Moreover, the first preparation method of the present invention includes a step for obtaining N-heterocyclicmethyl-alkylamine derivatives by use of a reductive amination reaction, in which aldehyde derivatives described by the following formula (IV):

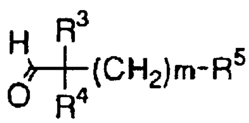

(IV)

and heterocyclicmethylamine derivatives described by the following formula (V):

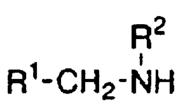

(V)

are reacted to form the following formula (I).

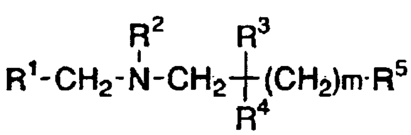

(I)

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind

selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively. The formula (II) is:

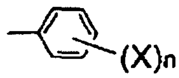

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

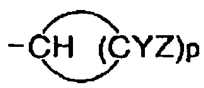

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III).]

Furthermore, the second preparation method of the present invention is characterized by obtaining N-heterocyclicmethyl-alkylamine derivatives by use of alkylamine derivatives described by the following formula (VI):

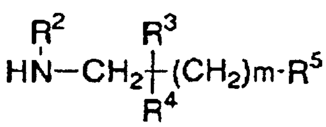

(VI)

and heterocyclicmethylation agents described by the following formula (VII):

$$R^1\text{-}CH_2\cdot W \qquad \text{(VII)}$$

to form the following formula (I):

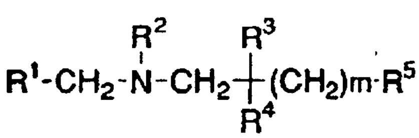

(I)

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated

alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively. The formula (II) is:

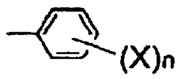

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

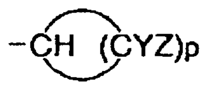

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III).]

In the aforementioned first method, it is preferable that the third preparation method of the present invention is employed. Namely, aldehyde derivatives are preferably used which are obtained by use of aldehyde derivatives described by the following formula (VIII):

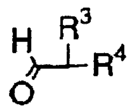

(VIII)

and alkylation agents described by the following formula (IX) :

$$W\text{-}(CH_2)m\cdot R^5 \qquad \text{(IX)}$$

to form the following formula (IV):

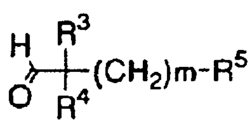

(IV)

[in the formula, $R^3$ represents one kind selected from a group consisting of an alkyl group having a carbon number of 1 to 5 and a halogenated alkyl group having a carbon number of 1 to 5; $R^4$ represents one kind selected from a group consisting of an alkyl group having a carbon number of 1 to 5 and a halogenated alkyl group having a carbon number of 1 to 5; carbon atoms in $R^3$ and $R^4$ may be bonded to form a ring structure; m represents an integer of 1 to 3; $R^5$ represents one kind selected from a group consisting of a phenyl group and a cycloalkyl group described by the following

formulae (II) and (III), respectively. The formula (II) is:

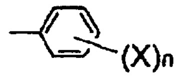 (II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

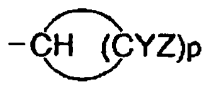 (III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.); and W represents a leaving group.]

Additionally, in the foregoing fist preparation method, it is preferable that the fourth preparation method of the present invention is employed. Namely, aldehyde derivatives are preferably used which are obtained by use of imine derivatives described by the following formula (X) :

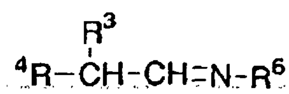 (X)

and alkylation agents described by the following formula (IX) :

$$W\text{-}(CH_2)m\cdot R^5 \qquad (IX)$$

to form the following formula (IV):

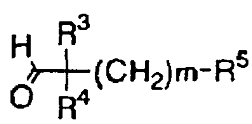 (IV)

[in the formula, $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively. The formula (II) is:

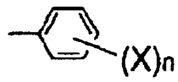

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6) , an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

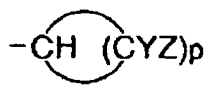

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III). $R^6$ represents one kind selected from a group consisting of an alkyl group having a carbon number of 2 to 6 and a cycloalkyl group having a carbon number of 3 to 6.]

Moreover, in the foregoing fist preparation method, it is preferable that the fifth preparation method of the present invention is employed. Namely, aldehyde derivatives are preferably used which are obtained by a reduction of ester derivatives described by the following formula (XI):

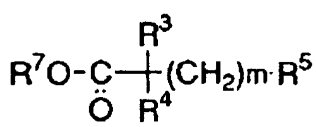

(XI)

to form the following formula (IV):

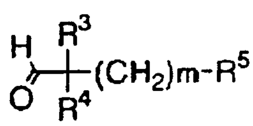

(IV)

[in the formula, $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively. The formula (II) is:

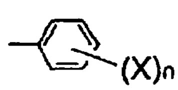

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and Beach represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

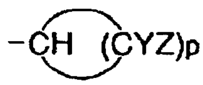

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III). $R^7$ represents an alkyl group having a carbon number of 1 to 3.]

Furthermore, in the foregoing second preparation method, it is preferable that the sixth preparation method of the present invention is employed. Namely, alkylamine derivatives are preferably used which are obtained by applying a reductive amination reaction using aldehyde derivatives described by the following'formula (IV):

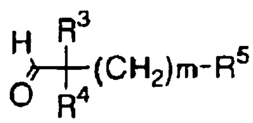

(IV)

and amination agents described by the following formula (XII) :

$R^2$-$NH_2$ **(XII)**

to form the following formula (VI):

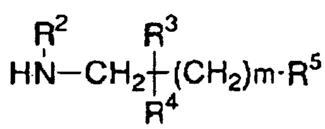

(VI)

[ in the formula, $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring'structure; m represents an integer of 1 to 3; $R^5$ represents one kind selected from a group consisting of a phenyl group and a cycloalkyl group described by the following formulae (II) and (III), respectively. The formula (II) is:

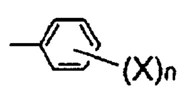

(II)

(in the formula, X represents one kind selected from a group consisting of a hydrogen atom, a halogen atom, an alkyl

group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6) , an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

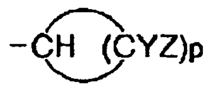

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III).]

Still furthermore, in the foregoing second preparation method, it is also preferable that the seventh preparation method of the present invention is employed. Namely, alkylamine derivatives are preferably used which are obtained by a reduction of alkylamide derivatives described by the following formula (XIII):

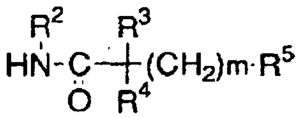

(XIII)

to form the following formula (VI):

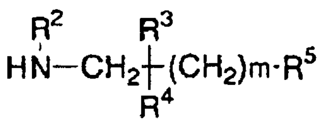

(VI)

[in the formula, $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively. The formula (II) is:

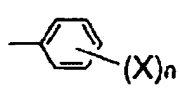

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6) , an acyl group, an ester group, a cyano

group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

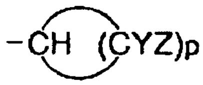 (III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may be each the same or different.) When m is 1 and $R^4$ is a hydrogen atom, $R^5$ is a cycloalkyl group described by the foregoing formula (III).]

In addition, fungicides of the present invention are characterized by containing N-heterocyclicmethyl-alkylamine derivatives described by the following general formula (I) or the acid addition salts thereof as an effective ingredient.

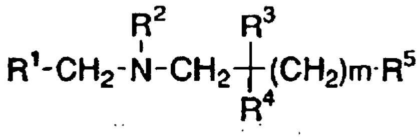 ( I )

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively. The formula (II) is:

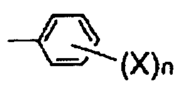 (II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

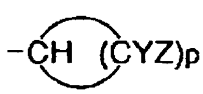 (III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6. The letter p represents an integer of 2 to 5, and the groups described by CYZ may

be each the same or different.) When m is 1 and $R^4$ is a hydrogen atom, $R^5$ is a cycloalkyl group described by the foregoing formula (III).]

**Best Mode for Carrying Out the Invention**

Hereinafter, preferable embodiments of the present invention will be explained in detail.

N-heterocyclicmethyl-alkylamine derivatives of the present invention are the compounds described by the following formula (I) or acid addition salts thereof.

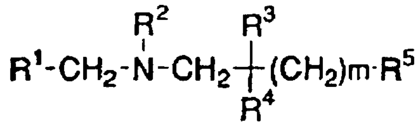

( I )

In the foregoing formula (I), $R^1$ represents a heterocycle which has at least one nitrogen atom as a hetero atom and may have a substituent on a ring. The above-described heterocycle has at least one nitrogen atom, however, furthermore, it may have other hetero atoms (an oxygen atom, a sulfur atom, and the like). Note that although the number of atoms constituting the foregoing heterocylce is not particularly limited, the heterocycle is preferably a 5- or 6-membered ring. Specific examples are preferably pyridine, pyrazine, pyrimidine, thiazole, triazole, imidazole, pyrazole or pyrrole. Among these, pyridine is particularly preferable which is bonded to amine at the 3-position on the ring through a methylene group.

Moreover, substituents included by the foregoing heterocycle are halogen atoms such as a fluorine atom, a chlorine atom, a bromine atom and an iodine atom; an alkyl group (preferably an alkyl group having a carbon number of 1 to 4, more preferably a methyl group, an ethyl group and a 1-methylethyl group); a halogenated alkyl group (preferably an fluorinatedalkyl group having a carbon number of 1 to 4, morepreferablyatrifluoromethyl group) ; analkoxy group (preferably an alkoxy group having a carbon number of 1 to 4, more preferably a methoxy group) ; a dialkylamino group (the carbon number of alkyl group is 1 to 4); a nitro group and the like. Here, the number of substituents included by the above-mentioned heterocycle and the bonding position thereof are not particularly limited. When the number of substituents is 2 or more, they may be each the same or different, however, the number of substituents is preferably 1 to 2, and the halogen atom is particularly preferable of the foregoing substituents. When the number of substituenst in the heterocycle is none or not less than 3, the fungicidal activity tends to decrease.

In the foregoing formula (I), $R^2$ represents a hydrogen atom or an alkyl group having a carbon number of 1 to 5; $R^3$ represents an alkyl group having a carbon number of 1 to 5 and a halogenated alkyl group having a carbon number of 1 to 5; $R^4$ represents a hydrogen atom, an alkyl group having a carbon number of 1 to 5, or a halogenated alkyl group having a carbon number of 1 to 5; and m represents an integer of 1 to 3. Here, $R^2$ is preferably a hydrogen atom or a methyl group. When $R^2$ is a hydrogen atom or a methyl group, higher fungicidal activity tends to be attained. Besides, $R^4$ is preferably a hydrogen atom, a methyl group or an ethyl group. When $R^4$ is a hydrogen atom, a methyl group or an ethyl group, higher fungicidal activity tends to be attained. Note that when $R^4$ is an alkyl group having a carbon number of 1 to 5 or a halogenated alkyl group having a carbon number of 1 to 5, carbon atoms in $R^3$ and $R^4$ may be bonded to form a ring structure.

In the foregoing formula (I), $R^5$ represents a phenyl group described by the following formula (II):

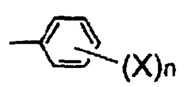

( II )

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) or a cycloalkyl group described by the following formula (III):

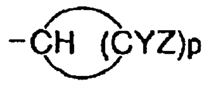

(III)

(in the formula, Y and Z may be each the same or different, and represent a hydrogen atom, an alkyl group having a carbon number of 1 to 6 or a halogenated alkyl group having a carbon number of 1 to 6, p represents an integer of 2 to 5, and the groups described by CYZ may be each the same or different.) However, in the foregoing formula (I), when m is 1 and $R^4$ is a hydrogen atom, $R^5$ is a cycloalkyl group described by the above-mentioned formula (III).

Herein, examples of X in the foregoing formula (II) are preferably halogen atoms such as a fluorine atom and a chlorine atom; a tertiary alkyl group such as a 1,1-dimethylethyl group (t-butyl group); a halogenated alkoxy group such as a trifluoromethoxy group; a phenoxy group such as a non-substituted phenoxy group; or a cycloalkyl group such as a cyclohexyl group. Further, the number n of these substituents is preferably 1 to 3 because higher fungicidal activity tends to be attained. Note that when n is 2 or more, X may be the same or different. In addition, X may be cross-linked each other to be condensed into a benzene ring forming a 5- or 6-membered ring. The preferable example of such condensed ring is a 2,2-difluoro-1,3-benzodioxol ring.

Moreover, in the foregoing formula (III), when p is 2 to 3, or when Y and Z are alkyl groups having a carbon number of 1 to 6 or halogenated alkyl groups having a carbon number of 1 to 6, and the number of substituents thereof is 2 or more, the fungicidal activity tends to decrease. In the present invention, it is preferable that p is 4 to 5, and when Y and Z represent alkyl groups having a carbon number of 1 to 6 or halogenated alkyl groups having a carbon number of 1 to 6, the number of substituents thereof is preferably 1. Such an example of a cycloalkyl group described by the foregoing formula (III) is a 4-(1,1-dimethylethyl)cyclohexyl group.

N-heterocyclicmethyl-alkylamine derivatives of the present invention which have the aforementioned structure exhibit the fungicidal activity on various pathogenic fungi. Thus, the preferable examples of such compounds are specifically cited as the compounds I-1 to I-142, which have structures shown in Table 1 to Table 7 wherein $R^1$, $R^2$, $R^3$, $R^4$, $R^5$ and m in the foregoing formula (I) are specified. Note that as for the compound I-26 described in Table 2, only compound name is written but not $R^1$, $R^2$, $R^3$, $R^4$, $R^5$ and m. I-26 is a compound in which carbon atoms of $R^3$ and $R^4$ in the formula (I) are bonded to form a ring structure.

Table 1

| Compound No. | $R^1$ | $R^2$ | $R^3$ | $R^4$ | m | $R^5$ |
|---|---|---|---|---|---|---|
| I-1 | 2-pyridyl | hydrogen atom | methyl | methyl | 1 | 4-(1,1-dimethylethyl)phenyl |
| I-2 | 6-chloro-3-pyridyl | hydrogen atom | methyl | methyl | 1 | 4-(1,1-dimethylethyl)phenyl |
| I-3 | 6-chloro-3-pyridyl | hydrogen atom | methyl | methyl | 1 | 2-chrolophenyl |
| I-4 | 2-pyrazyl | hydrogen atom | methyl | methyl | 1 | 4-(1,1-dimethylethyl)phenyl |
| I-5 | 5-chrolo-2-pyrazyl | hydrogen atom | methyl | methyl | 1 | 4-(1,1-dimethylethyl)phenyl |
| I-6 | 1-methyl-1H-pyrazol-4-yl | hydrogen atom | methyl | methyl | 1 | 4-(1,1-dimethylethyl)phenyl |
| I-7 | 1-methyl-1H-pyrazol-4-yl | hydrogen atom | methyl | methyl | 1 | 2-fluorophenyl |
| I-8 | 1-methyl-1H-pyrazol-4-yl | hydrogen atom | methyl | methyl | 1 | 4-fluorophenyl |
| I-9 | 1-methyl-1H-pyrazol-4-yl | hydrogen atom | methyl | methyl | 1 | 2,4-difluorophenyl |
| I-10 | 4-pyrimidyl | hydrogen atom | methyl | methyl | 1 | 4-(1,1-dimethylethyl)phenyl |
| I-11 | 1H-imidazol-4-yl | hydrogen atom | methyl | methyl | 1 | 4-(1,1-dimethylethyl)phenyl |
| I-12 | 1H-imidazol-4-yl | hydrogen atom | methyl | methyl | 1 | 2,4-difluorophenyl |
| I-13 | 1H-pyrrolyl | hydrogen atom | methyl | methyl | 1 | 4-(1,1-dimethylethyl)phenyl |

Table 2

| Compound No. | $R^1$ | $R^2$ | $R^3$ | $R^4$ | m | $R^5$ |
|---|---|---|---|---|---|---|
| I-14 | 2-chloro-5-thiazolyl | methyl | methyl | methyl | 1 | 4-(1,1-dimethylethyl)phenyl |
| I-15 | 2-pyridyl | methyl | methyl | methyl | 1 | 4-(1,1-dimethylethyl)phenyl |
| I-16 | 3-pyridyl | methyl | methyl | methyl | 1 | 4-(1,1-dimethylethyl)phenyl |
| I-17 | 4-pyridyl | methyl | methyl | methyl | 1 | 4-(1,1-dimethylethyl)phenyl |
| I-18 | 6-chloro-3-pyridyl | methyl | methyl | methyl | 1 | 4-(1,1-dimethylethyl)phenyl |
| I-19 | 6-fluoro-3-pyridyl | methyl | methyl | methyl | 1 | 4-(1,1-dimethylethyl)phenyl |
| I-20 | 2-chloro-3-pyridyl | methyl | methyl | methyl | 1 | 4-(1,1-dimethylethyl)phenyl |
| I-21 | 2-methyl-5-pyrimidyl | methyl | methyl | methyl | 1 | 4-(1,1-dimethylethyl)phenyl |
| I-22 | 2-pyrazyl | methyl | methyl | methyl | 1 | 4-(1,1-dimethylethyl)phenyl |
| I-23 | 1,2,4-triazol-1-yl | methyl | methyl | methyl | 1 | 4-(1,1-dimethylethyl)phenyl |
| I-24 | 1-methyl-2-pyrrolyl | methyl | methyl | methyl | 1 | 4-(1,1-dimethylethyl)phenyl |
| I-25 | 6-chrolo-3-pyridyl | methyl | methyl | methyl | 1 | 4-(1,1-dimethylethyl)phenyl |
| I-26 | N-{[1-(4-(1,1-dimethylethyl)benzyl)cyclobutyl]methyl}-6-chloro-N-methyl-3-pyridylmethylamine | | | | | |

Table 3

| Compound No. | $R^1$ | $R^2$ | $R^3$ | $R^4$ | m | $R^5$ |
|---|---|---|---|---|---|---|
| I-27 | 6-chloro-3-pyridyl | methyl | methyl | methyl | 1 | 3-(1-methylethoxy)phenyl |
| I-28 | 6-chloro-3-pyridyl | methyl | methyl | methyl | 1 | 4-chlorophenyl |
| I-29 | 6-chloro-3-pyridyl | methyl | methyl | methyl | 1 | 3-chlorophenyl |
| I-30 | 6-chloro-3-pyridyl | methyl | methyl | methyl | 1 | 2-chlorophenyl |
| I-31 | 6-chloro-3-pyridyl | methyl | methyl | methyl | 1 | 2,4-dichlorophenyl |
| I-32 | 6-chloro-3-pyridyl | methyl | methyl | methyl | 1 | 2,5-dichlorophenyl |
| I-33 | 6-chloro-3-pyridyl | methyl | methyl | methyl | 1 | 3,4-dichlorophenyl |
| I-34 | 6-chloro-3-pyridyl | methyl | methyl | methyl | 1 | 3,5-dichlorophenyl |
| I-35 | 6-chloro-3-pyridyl | methyl | methyl | methyl | 1 | 4-phenoxyphenyl |
| I-36 | 6-chloro-3-pyridyl | methyl | methyl | methyl | 1 | 3-phenoxyphenyl |
| I-37 | 6-chloro-3-pyridyl | methyl | methyl | methyl | 1 | 2-phenoxyphenyl |
| I-38 | 6-chloro-3-pyridyl | methyl | methyl | methyl | 1 | 3-phenoxy-4-fluorophenyl |
| I-39 | 6-chloro-3-pyridyl | methyl | methyl | methyl | 1 | 3-(4-methylphenoxy)phenyl |
| I-40 | 6-chloro-3-pyridyl | methyl | methyl | methyl | 1 | 3-(3-trifluoromethylphenoxy)phenyl |
| I-41 | 6-chloro-3-pyridyl | methyl | methyl | methyl | 1 | 3-biphenyl |
| I-42 | 6-chloro-3-pyridyl | methyl | methyl | methyl | 1 | 3-trifluoromethylphenyl |
| I-43 | 6-chloro-3-pyridyl | methyl | methyl | methyl | 1 | 4-fluoro-3-trifluoromethylphenyl |
| I-44 | 6-chloro-3-pyridyl | methyl | methyl | methyl | 1 | 2,4-bis(trifluoromethyl)phenyl |
| I-45 | 6-chloro-3-pyridyl | methyl | methyl | methyl | 1 | 4-trifluoromethoxyphenyl |
| I-46 | 6-chloro-3-pyridyl | methyl | methyl | methyl | 1 | 3-trifluoromethoxyphenyl |
| I-47 | 6-chloro-3-pyridyl | methyl | methyl | methyl | 1 | 2-trifluoromethoxyphenyl |
| I-48 | 6-chloro-3-pyridyl | methyl | methyl | methyl | 1 | 3-(1,1,2,2-tetrafluoroethoxy)phenyl |
| I-49 | 6-chloro-3-pyridyl | methyl | methyl | methyl | 1 | 2,2-difluoro-1,3-benzodioxol-5-yl |

Table 4

| Compound No. | $R^1$ | $R^2$ | $R^3$ | $R^4$ | m | $R^5$ |
|---|---|---|---|---|---|---|
| I-50 | 6-chloro-3-pyridyl | methyl | methyl | hydrogen atom | 2 | 4-(1,1-dimethylethyl)phenyl |
| I-51 | 6-chloro-3-pyridyl | methyl | methyl | methyl | 2 | 4-(1,1-dimethylethyl)phenyl |
| I-52 | 6-chloro-3-pyridyl | methyl | methyl | hydrogen atom | 3 | 4-(1,1-dimethylethyl)phenyl |
| I-53 | 6-chloro-3-pyridyl | methyl | methyl | methyl | 3 | 4-(1,1-dimethylethyl)phenyl |
| I-54 | 6-chloro-3-pyridyl | methyl | methyl | hydrogen atom | 2 | 3-phenoxyphenyl |
| I-55 | 6-chloro-3-pyridyl | methyl | methyl | methyl | 2 | 3-phenoxyphenyl |
| I-56 | 6-chloro-3-pyridyl | methyl | methyl | hydrogen atom | 2 | 2,4-dichlorophenyl |
| I-57 | 6-chloro-3-pyridyl | methyl | methyl | hydrogen atom | 2 | 3,5-dichlorophenyl |
| I-58 | 6-chloro-3-pyridyl | methyl | methyl | hydrogen atom | 2 | 3-trifluoromethoxyphenyl |
| I-59 | 6-chloro-3-pyridyl | methyl | methyl | methyl | 2 | 3-trifluoromethoxyphenyl |
| I-60 | 6-chloro-3-pyridyl | methyl | methyl | hydrogen atom | 2 | 4-trifluoromethoxyphenyl |
| I-61 | 6-chloro-3-pyridyl | methyl | methyl | methyl | 2 | 4-trifluoromethoxyphenyl |
| I-62 | 6-chloro-3-pyridyl | methyl | methyl | hydrogen atom | 1 | 4-(1,1-dimethylethyl)cyclohexyl |
| I-63 | 6-chloro-3-pyridyl | methyl | methyl | methyl | 1 | 4-(1,1-dimethylethyl)cyclohexyl |
| I-64 | 6-chloro-3-pyridyl | methyl | methyl | hydrogen atom | 2 | 4-(1,1-dimethylethyl)cyclohexyl |
| I-65 | 6-chloro-3-pyridyl | methyl | methyl | methyl | 2 | 4-(1,1-dimethylethyl)cyclohexyl |
| I-66 | 6-chloro-3-pyridyl | methyl | methyl | hydrogen atom | 3 | 4-(1,1-dimethylethyl)cyclohexyl |
| I-67 | 6-chloro-3-pyridyl | methyl | methyl | methyl | 3 | 4-(1,1-dimethylethyl)cyclohexyl |
| I-68 | 6-chloro-3-pyridyl | methyl | methyl | methyl | 1 | 4-methoxyphenyl |
| I-69 | 6-chloro-3-pyridyl | methyl | methyl | methyl | 1 | 3-methoxyphenyl |
| I-70 | 6-chloro-3-pyridyl | methyl | methyl | methyl | 1 | 2-methoxyphenyl |
| I-71 | 6-chloro-3-pyridyl | methyl | methyl | methyl | 1 | 4-cyclopropylmethoxyphenyl |
| I-72 | 6-chloro-3-pyridyl | methyl | methyl | methyl | 1 | 3-cyclopropylmethoxyphenyl |

Table 5

| Compound No. | $R^1$ | $R^2$ | $R^3$ | $R^4$ | m | $R^5$ |
|---|---|---|---|---|---|---|
| I-73 | 6-chloro-3-pyridyl | methyl | methyl | methyl | 1 | 4-(1,1-dimethylethoxy)phenyl |
| I-74 | 6-chloro-3-pyridyl | methyl | methyl | methyl | 1 | 4-(1-methylethoxy)phenyl |
| I-75 | 6-chloro-3-pyridyl | methyl | methyl | methyl | 1 | 4-(2-methylpropoxy)phenyl |
| I-76 | 6-chloro-3-pyridyl | methyl | methyl | methyl | 1 | 4-(1-methylpropoxy)phenyl |
| I-77 | 6-chloro-3-pyridyl | methyl | methyl | methyl | 1 | 4-allyloxyphenyl |
| I-78 | 6-chloro-3-pyridyl | methyl | methyl | methyl | 1 | 4-(2-propynyloxy)phenyl |
| I-79 | 6-chloro-3-pyridyl | methyl | methyl | methyl | 1 | 3-cyclohexyloxyphenyl |
| I-80 | 6-chloro-3-pyridyl | methyl | methyl | methyl | 1 | 3-benzyloxyphenyl |
| I-81 | 6-chloro-3-pyridyl | methyl | methyl | methyl | 1 | 4-trimethylphenylmethoxyphenyl |
| I-82 | 6-chloro-3-pyridyl | methyl | methyl | methyl | 1 | 4-(2,2,2-trifluoroethoxy)phenyl |
| I-83 | 6-chloro-3-pyridyl | methyl | methyl | methyl | 1 | 4-(1,1,2,2-tetrafluoroethoxy)phenyl |
| I-84 | 6-chloro-3-pyridyl | methyl | methyl | methyl | 1 | 4-(2,2,3,3-tetrafluoropropoxy)phenyl |
| I-85 | 6-chloro-3-pyridyl | methyl | methyl | methyl | 1 | 4-(2,2,3,3,3-pentafluoropropoxy)phenyl |
| I-86 | 6-chloro-3-pyridyl | methyl | methyl | methyl | 1 | 4-(1,1,2,3,3,3-hexafluoropropoxy)phenyl |
| I-87 | 6-chloro-3-pyridyl | methyl | methyl | methyl | 1 | 4-hydroxymethylphenyl |
| I-88 | 6-chloro-3-pyridyl | methyl | methyl | methyl | 1 | 4-methoxymethylphenyl |
| I-89 | 6-chloro-3-pyridyl | methyl | methyl | methyl | 1 | 4-ethoxymethylphenyl |
| I-90 | 6-chloro-3-pyridyl | methyl | methyl | methyl | 1 | 4-(1-methylethoxymethyl)phenyl |
| I-91 | 6-chloro-3-pyridyl | methyl | methyl | methyl | 1 | 4-cyclopropylmethoxymethylphenyl |
| I-92 | 6-chloro-3-pyridyl | methyl | methyl | methyl | 1 | 4-(2,2,2-trifluoroethoxymethyl)phenyl |
| I-93 | 6-chloro-3-pyridyl | methyl | methyl | methyl | 1 | 4-[(2,2,2-trifluoro-1-trifluoromethylethoxy)methyl]phenyl |
| I-94 | 6-chloro-3-pyridyl | methyl | methyl | methyl | 1 | 4-(1-hydroxyethyl)phenyl |
| I-95 | 6-chloro-3-pyridyl | methyl | methyl | methyl | 1 | 4-(1-methoxyethyl)phenyl |
| I-96 | 6-chloro-3-pyridyl | methyl | methyl | methyl | 1 | 4-(1-ethoxyethyl)phenyl |

Table 6

| Compound No. | $R^1$ | $R^2$ | $R^3$ | $R^4$ | m | $R^5$ |
|---|---|---|---|---|---|---|
| I-97 | 6-chloro-3-pyridyl | methyl | methyl | methyl | 1 | 4-(1-cyclopropylmethoxyethyl)phenyl |
| I-98 | 6-chloro-3-pyridyl | methyl | methyl | methyl | 1 | 4-(1-hydroxy-1-methylethylphenyl) |
| I-99 | 6-chloro-3-pyridyl | methyl | methyl | methyl | 1 | 4-(1-methoxy-1-methylethyl)phenyl |
| I-100 | 6-chloro-3-pyridyl | methyl | methyl | methyl | 1 | 4-(1-ethoxy-1-methylethyl)phenyl |
| I-101 | 6-chloro-3-pyridyl | methyl | methyl | methyl | 1 | 4-{1-(2,2,2-trifluoro-2-trifluoromethylethoxy) ethyl}phenyl |
| I-102 | 6-chloro-3-pyridyl | methyl | methyl | methyl | 1 | 4-formylphenyl |
| I-103 | 6-chloro-3-pyridyl | methyl | methyl | methyl | 1 | 4-acetylphenyl |
| I-104 | 6-chloro-3-pyridyl | methyl | methyl | methyl | 1 | 3-acetylphenyl |
| I-105 | 6-chloro-3-pyridyl | methyl | methyl | methyl | 1 | 4-propionylphenyl |
| I-106 | 6-chloro-3-pyridyl | methyl | methyl | methyl | 1 | 3-(3-methylbutyryl)phenyl |
| I-107 | 6-chloro-3-pyridyl | methyl | methyl | methyl | 1 | 4-(3-oxobutyryl)phenyl |
| I-108 | 6-chloro-3-pyridyl | methyl | methyl | methyl | 1 | 3-benzoylphenyl |
| I-109 | 6-chloro-3-pyridyl | methyl | methyl | methyl | 1 | 4-(2-methyl-1,3-dioxolane-2-yl)phenyl |
| I-110 | 6-chloro-3-pyridyl | methyl | methyl | methyl | 1 | 4-(2-methyl-1,3-dioxolane-2-yl)phenyl |
| I-111 | 6-chloro-3-pyridyl | methyl | methyl | methyl | 1 | 4-(1,5-dimethylpyrazol-3-yl)phenyl |
| I-112 | 6-chloro-3-pyridyl | methyl | methyl | methyl | 1 | 4-(5-methyl-1,2,4-oxadiazol-3-yl)phenyl |
| I-113 | 6-chloro-3-pyridyl | methyl | methyl | methyl | 1 | 4-(5-trifluoromethyl-1,2,4-oxadiazol-3-yl)phen yl |
| I-114 | 6-chloro-3-pyridyl | methyl | methyl | methyl | 1 | 4-(1-amino-1-hydroxyiminomethyl)phenyl |
| I-115 | 6-chloro-3-pyridyl | methyl | methyl | methyl | 1 | 4-(1-hydroxyiminoethyl)phenyl |
| I-116 | 6-chloro-3-pyridyl | methyl | methyl | methyl | 1 | 4-(1-methoxyiminoethyl)phenyl |
| I-117 | 6-chloro-3-pyridyl | methyl | methyl | methyl | 1 | 4-(1-ethoxyiminoethyl)phenyl |
| I-118 | 6-chloro-3-pyridyl | methyl | methyl | methyl | 1 | 4-[1-(1-methylethoxy)iminoethyl]phenyl |
| I-119 | 6-chloro-3-pyridyl | methyl | methyl | methyl | 1 | 4-(1-cyclopropylmethoxyiminoethyl)phenyl |

Table 7

| Compound No. | $R^1$ | $R^2$ | $R^3$ | $R^4$ | m | $R^5$ |
|---|---|---|---|---|---|---|
| I-120 | 6-chloro-3-pyridyl | methyl | methyl | methyl | 1 | 4-[1-(2,2,2-trifluoroethoxyimino)ethyl]phenyl |
| I-121 | 6-chloro-3-pyridyl | methyl | methyl | methyl | 1 | 4-(1-methoxyiminopropyl)phenyl |
| I-122 | 6-chloro-3-pyridyl | methyl | methyl | methyl | 1 | 4-(1-ethoxyiminopropyl)phenyl |
| I-123 | 6-chloro-3-pyridyl | methyl | methyl | methyl | 1 | 4-(1-methoxyimino-2-methylpropyl)phenyl |
| I-124 | 6-chloro-3-pyridyl | methyl | methyl | methyl | 1 | 3-methoxyiminomethylphenyl (isomer 1) |
| I-125 | 6-chloro-3-pyridyl | methyl | methyl | methyl | 1 | 3-ethoxyiminomethylphenyl (isomer 2) |
| I-126 | 6-chloro-3-pyridyl | methyl | methyl | methyl | 1 | 3-(1-methoxyiminoethyl)phenyl |
| I-127 | 6-chloro-3-pyridyl | methyl | methyl | methyl | 1 | 3-(1-ethoxyiminoethyl)phenyl |
| I-128 | 6-chloro-3-pyridyl | methyl | methyl | methyl | 1 | 3-[1-(1-methylethoxy)iminoethyl]phenyl |
| I-129 | 6-chloro-3-pyridyl | methyl | methyl | methyl | 1 | 3-(1-methoxyimino-2-methylpropyl)phenyl |
| I-130 | 6-chloro-3-pyridyl | methyl | methyl | methyl | 1 | 4-cyanophenyl |
| I-131 | 6-chloro-3-pyridyl | methyl | methyl | methyl | 1 | 3-cyanophenyl |
| I-132 | 6-chloro-3-pyridyl | methyl | methyl | methyl | 1 | 3-(1,1-dimethylethoxycarbonyl)phenyl |
| I-133 | 6-chloro-3-pyridyl | methyl | methyl | methyl | 1 | 3-benzylphenyl |
| I-134 | 6-chloro-3-pyridyl | methyl | methyl | methyl | 1 | 4-trifluoromethylphenyl |
| I-135 | 6-chloro-3-pyridyl | methyl | methyl | methyl | 1 | 4-vinylphenyl |
| I-136 | 6-chloro-3-pyridyl | methyl | methyl | methyl | 1 | 4-(1-methylvinyl)phenyl |
| I-137 | 6-chloro-3-pyridyl | methyl | methyl | methyl | 1 | 4-trifluoromethoxyphenyl |
| I-138 | 3-pyridyl | methyl | methyl | methyl | 1 | 4-trifluoromethoxyphenyl |
| I-139 | 6-bromo-3-pyridyl | methyl | methyl | methyl | 1 | 4-trifluoromethoxyphenyl |
| I-140 | 6-fluoro-3-pyridyl | methyl | methyl | methyl | 1 | 4-trifluoromethoxyphenyl |
| I-141 | 6-trifluoromethyl-3-pyridyl | methyl | methyl | methyl | 1 | 4-trifluoromethoxyphenyl |
| I-142 | 2-chloro-5-thiazolyl | methyl | methyl | methyl | 1 | 4-trifluoromethoxyphenyl |

I-124 and I-125 are geometrical isomers with respect to the imino group on $R^5$.

The N-heterocyclicmetyl-alkylamine derivatives of the present invention described by the foregoing general

formula (I) are obtained by a method shown by the following reaction scheme (A):

Reaction scheme (A):

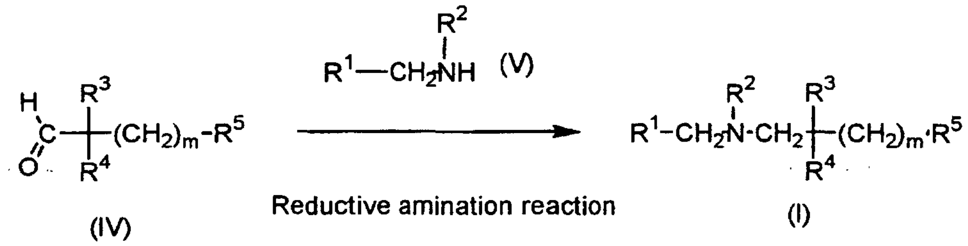


namely, aldehyde derivatives (IV) and heterocyclicmethylamine derivatives (V) are reduced by applying a reductive amination reaction in the presence of a reducing agent, or by a method shown by the following reaction scheme (B):

Reaction scheme (B):

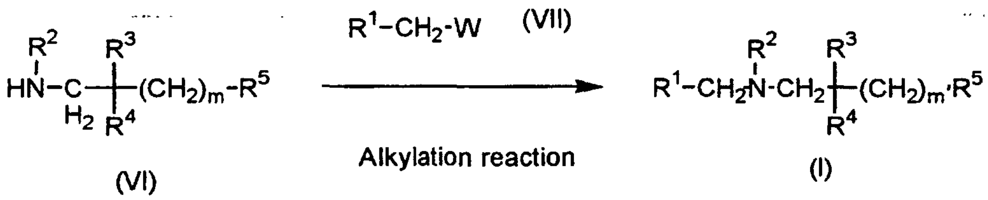


namely, the nitrogen atom in amino group of alkylamine derivatives (VI) is subjected to an alkylation by use of a heterocyclicmethylation agent (VII). Note that in the scheme, $R^1$, $R^2$, $R^3$, $R^4$, $R^5$ and m have the same definitions as those described above, and W represents a leaving group.

Herein, as shown in the following reaction scheme (C) :

Reaction scheme (C):

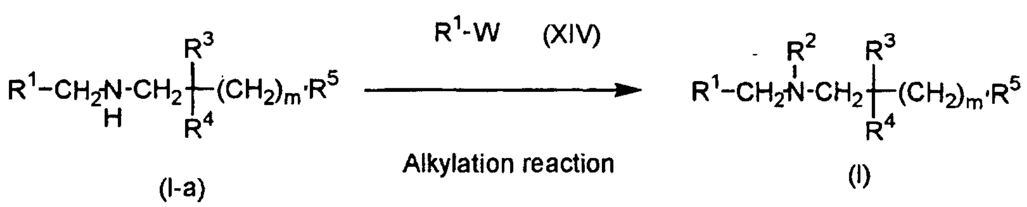


N-heterocyclicmethyl-alkylamine derivatives (I-b) of tertiary amine can be obtained through an alkylation of N-heterocyclicmethyl-alkylamine derivatives (I-a) of secondary amine by use of an alkylation agent (XIV). Note that in the scheme, $R^1$, $R^3$, $R^4$, $R^5$ andmhave the same definitions as those described above, $R^2$ represents an alkyl group having a carbon number of 1 to 5, and W represents a leaving group.

In the methods described by the foregoing reaction schemes (A) and (B), aldehyde derivatives (IV) of starting raw material can be synthesized by applying the method written in the following reference:

Tetrahedron Lett., 15, 1273 (1973)

which gives a method to make an alkylation of the α carbon atom of aldehyde described in the following reaction scheme (D) :

Reaction scheme (D):

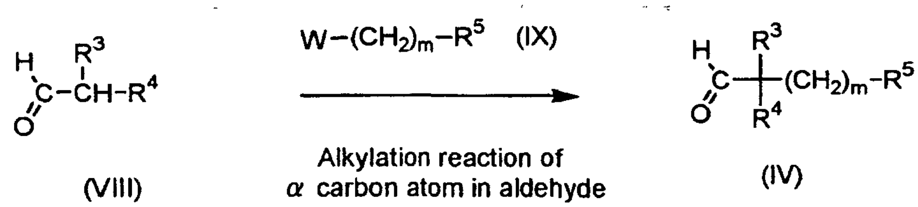


(in the scheme, $R^3$, $R^5$ and m have the same definitions as those described above, R4 represents an alkyl group having a carbon number of 1 to 5 or a halogenated alkyl group having a carbon number of 1 to 5, and W represents a leaving group.) Namely, aldehyde derivatives (VIII) and an alkylation agent (IX) are reacted to synthesize aldehyde derivatives (IV). Moreover, aldehyde derivatives (IV) are efficiently synthesized by using a method written in the following references:

Tetrahedron Lett., 8, 597 (1976); ibid, 17, 1379 (1976),

which give a method described in the following reaction scheme (E):

Reaction scheme (E):

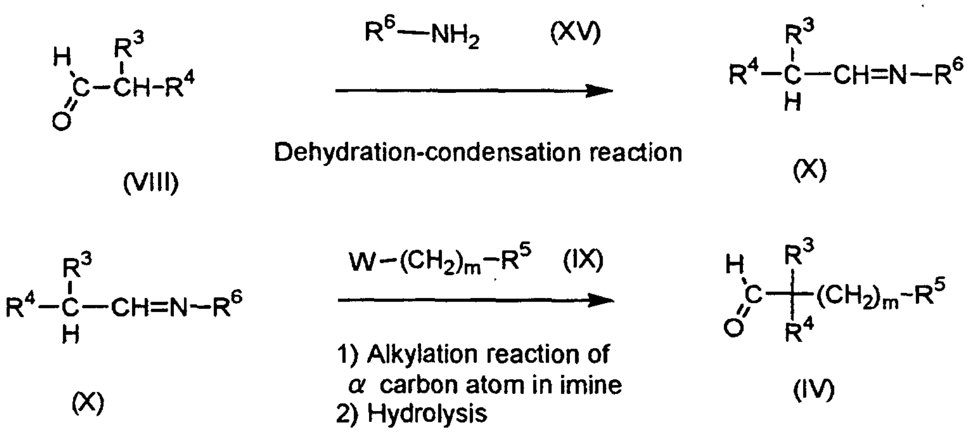


(in the scheme, $R^3$, $R^4$, $R^5$ and m have the same definitions as those described above, $R^6$ represents an alkyl group having a carbon number of 2 to 6 or a cycloalkyl group having a carbon number of 3 to 6, and W represents a leaving group.) . Namely, imine derivatives (X) is obtained through a dehydration-condensation of aldehyde derivatives (VIII) and alkylamine derivatives (XV), thereafter, the α carbon atom of imine derivatives (X) is alkylated by use of an alkylation agent (IX), and further hydrolyzed to efficiently form aldehyde derivatives (IV). Furthermore, ester derivatives (XI) obtained by the method written in Publication WO99/12902 are subjected to reduction through a method described in the following reaction scheme (F):

Reaction scheme (F):

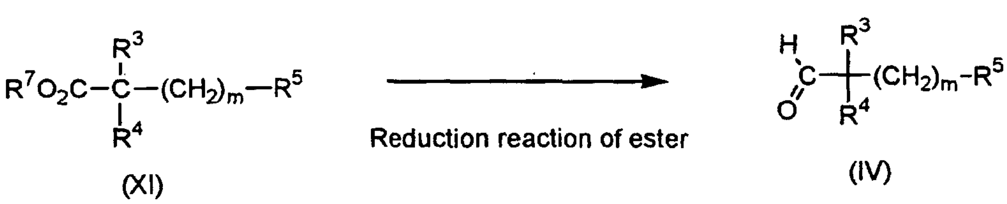

(in the scheme, $R^3$, $R^4$, $R^5$ and m have the same definitions as those described above, and $R^7$ represents an alkyl group having a carbon number of 1 to 3.).
Namely, aldehyde derivatives (IV) can be also obtained through a reduction using diisobutylaluminum hydride (DIBAL-H) .

In the method described by the foregoing reaction scheme (B), alkylamine derivatives (VI) can be obtained through a method shown in the following reaction scheme (G) :

Reaction scheme (G):

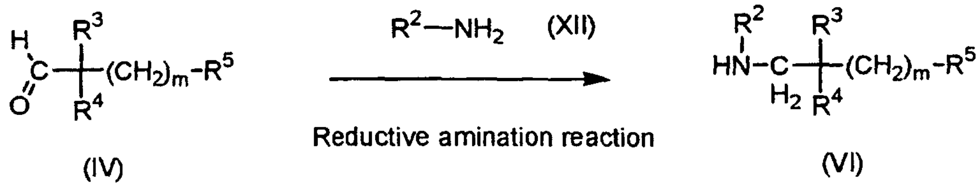


(IV) (VI)

(in the scheme, $R^2$, $R^3$, $R^4$, $R^5$ and m have the same definitions as those described above.). Namely, it can be obtained by applying a reductive amination reaction with aldehyde derivatives (IV) and an amination agent (XII) in the presence of a reducing agent. In addition, alkylamine derivatives (VI) can be also obtained by reacting alkylamide derivatives (XIII) which is synthesized through the method written in the gazette WO99/12902 using a scheme shown in the following reaction scheme (H):

Reaction scheme (H):

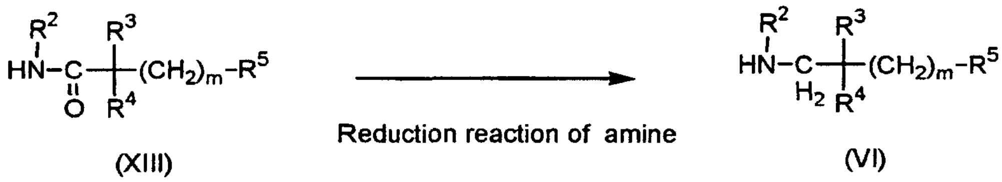


(XIII) (VI)

(in the scheme, $R^2$, $R^3$, $R^4$, $R^5$ and m have the same definitions as those described above.). Namely, it can be obtained through a reduction using lithium aluminium hydride.

In the foregoing method, commercially available products can be employed for some of heterocyclicmethylamine derivativs (V), an amination agent (XII), a heterocyclicmethylation agent (VII), an alkylation agent (IX), and an alkylation agent having a carbon number of 1 to 5 (XIV). Besides, these compounds can be synthesized by methods written in the following references:

J.Heterocyclic.Chem., 5, 407 (1968); ibid., 6, 549 (1969);
J. Org. Chem., 21, 97(1956);
Japanese Unexamined Patent Publication No. SHO 59-59669; Japanese Unexamined Patent Publication No. HEI 2-171;
Aust.J.Chem., 27, 2251(1974); and Chem.Pharm.Bull., 28, 3057 (1980).

Herein, the examples of aforementioned heterocyclicmethylamine derivatives (V) are 2-pyridylmethylamine, 3-pyridylmethylamine, 4-pyridylmethylamine, 6-chloro-3-pyridylmethylamine, 2-chloro-3-pyridylmethylamine, 6-fluoro-3-pyridylmethylamine, 6-bromo-3-pyridylmethylamine, 6-trifluoromethyl-3-pyridylmethylamine, 2-pyrazylmethylamine, 5-chloro-2-pyrazylmethylamine, 4-pyrimidylmethylamine, 2-methyl-5-pyrimidylmethylamine, 1-methyl-1H-pyrazol-4-ylmethylamine, 1H-imidazol-4-ylmethylamine, 1H-pyrrol-2-ylmethylamine, 1-methyl-1H-pyrral-2-ylmethylamine, 1,2,4-triazol-1-ylamine, 2-chloro-5-thiazolylmethylamine, N-methyl-2-pyridylmethylamine, N-methyl-3-pyridylmethylamine, N-methyl-4-pyridylmethylamine, 6-chloro-N-methyl-3-pyridylmethylamine, 2-chloro-N-methyl-3-pyridylmethylamine, 6-fluoro-N-methyl-3-pyridylmethylamine, 6-bromo-N-methyl-3-pyridylmethylamine, N-methyl-6-trifluoromethyl-3-pyridylmethylamine, N-methyl-2-pyrazylmethylamine, 5-chloro-N-methyl-2-pyrazylmethylamine, N-methyl-4-pyrimidylmethylamine, N,2-dimethyl-5-pyrimidylmethylamine, N,1-dimethyl-1H-pyrazol-4-ylmethylamine, N-methyl-1H-imidazol-4-ylmethylamine, N-methyl-1H-pyrrol-2-ylmethylamine, N,1-dimethyl-1H-pyrrol-2-ylmethylamine, N-methyl-1,2,4-triazol-1-ylamine, 2-chloro-N-methyl-5-thiazolylmethylamine, and the like;

The examples of amination agents (XII) are ammonia, methylamine, ethylamine, propylamine, isopropylamine, butylamine, isobutylamine, sec-butylamine, tert-butylamine, pentylamine, isopentylamine, and the like;

Th examples of heterocyclicalkylation agents (VII) are 2-chloromethylpyridine, 3-chloromethylpyridine, 4-chloromethylpyridine, α,6-dichloro-3-methylpyridine (same as 6-chloro-3-chloromethylpyridine), α,2-dichloro-3-methylpyridine (same as 2-chloro-3-chloromethylpyridine), 3-chloromethyl-6-fluoropyridine, 6-bromo-3-chloromethylpyridine, 3-chloromethyl-6-trifluoromethylpyridine, 2-chloromethylpyrazine, α,5-dichloro-2-methylpyrazine (same as 5-chloro-2-chloromethylpyrazine), 4-chloromethylpyrimidine, 5-chloromethyl-2-methylpyrimidine, 4-chloromethyl-1-methyl-1H-pyrazole, 4-chloromethyl-1H-imidazole, 2-chloromethyl-1H-pyrrole, 2-chloromethyl-1-methyl-1H-pyrrole, 1-chloromethyl-1,2,4-triazole, 2-chloro-5-chloromethylthiazole, and the like;

The examples of alkylation agents (IX) are 2-chlorobenzylchloride, 3-chlorobenzylchloride, 4-chlorobenzylchloride, 2,4-dichlorobenzylbromide, 2,5-dichlorobenzylbromide, 3,4-dichlorobenzylbromide, 3,5-dichlorobenzylchloride, 2-fluorobenzylchloride, 4-fluorobenzylchloride, 2,4-difluorobenzylchloride, 4-tert-butylbenzylbromide, 3-isopropoxybenzylbromide, 4-phenoxybenzylbromide, 3-phenoxybenzylbromide, 2-phenoxybenzylbromide, 3-(4-methylphenyl)oxybenzylbromide, 3-(3-trifluoromethylphenyl)oxybenzylbromide, 4-fluoro-3-phenoxybenzylbromide, 3-phenylbenzylbromide, 3-trifluoromethylbenzylbromide, 4-fluoro-3-trifluoromethylbenzylbromide, 2,4-bis(trifluoromethyl)benzylbromide, 4-trifluoromethoxybenzylbromide, 3-trifluoromethoxybenzylbromide, 2-trifluoromethoxybenzylbromide, 3-(1,1,2,2-tetrafluoroethoxy)benzylbromide, 2,2-difluoro-5-benzodioxolylmethylbromide, 4-tert-butylcyclohexylmethylbromide, 4-tert-butylphenethylbromide, 3-phenoxyphenethylbromide, 2,4-dichlorophenethylbromide, 3,5-dichlorophenethylbromide, 4-trifluoromethoxyphenethylbromide, 3-trifluoromethoxyphenethylbromide, 3-(4-tert-butylphenyl)propylbromide, 2-(4-tert-butylcyclohexyl)ethylbromide, 3-(4-tert-butylcyclohexyl)propylbromide, and the like;

The examples of alkylation agents (XIV) are methyliodide, methylbromide, ethyliodide, ethylbromide, isopropyliodide, isopropylbromide, butyliodide, isobutyliodide, sec-butyliodide, tert-butyliodide, pentyliodide, isopentyliodide, dimethylsulfuric acid, diethylsulfuric acid, methylp-toluenesulfonate, and the like.

Moreover, alkylation agents (IX), heterocyclicmethylation agents (VII) and alkylation agents having a carbon number of 1 to 5 (XIV) have leaving groups W. The examples of such compounds are a halogenation agent, sulfate ester, (non-substituted or substituted benzene)sulfonate, and the like. Herein, the preferable examples of leaving group W in these compounds are halogen atoms such as chlorine, bromine and iodine, and a p-toluenesulfonyloxy group.

(Reductive amination reaction)

As described above, in the preparation methods of N-heterocyclicmethyl-alkylamine derivatives of the present invention, a reductive amination reaction is applied in the synthesis steps for obtaining desired compounds or intermediates thereof. Such reductive amination reaction canbe conductedbyuse of themethods written in the following references:

J. Am. Chem. Soc., 93, 2897(1971);
Synthesis, 135(1975);
Org. React., 4, 174(1948);
J. Org. Chem., 61, 3849(1996); and
Tetrahedron Letters, 31, 5595(1990).

Herein, the reducing agents used in the foregoing reductive amination reaction are preferably complex hydride compounds such as sodium triacetoxyborohydride and sodium cyanoborohydride. Besides, other than the complex hydride compounds, suitably used are the combinations of hydrogenation catalysts such as hydrogen gas and palladium/charcoal and Raney nickel, formic acid.

Further, the foregoing reductive amination reaction may be conducted either in a solvent or in a solvent-free condition. Note that the examples of solvents used for the reaction in the solvent are alcohols such as methanol and ethanol; ethers such as tetrahydrofuran and dioxane; hydrocarbon halides such as 1,2-dichloroethane; water; and acetonitrile. These solvents may be used alone, or used in a mixed solvent which contains at least one kind thereof.

Furthermore, the reaction condition of the foregoing reductive amination reaction is not particularly limited, however, the amount of the foregoing reducing agent is preferably 1.0 to 20.0 times in mole compared with that of aldehyde derivatives (IV), more preferably 1.0 to 3.0 times in mole. When the amount of the reducing agent is less than 1.0 times in mole, the reaction yield tends to decrease. In addition, when the amount of the reducing agent exceeds 20.0 times in mole, the reaction gives a tendency of excess amount of the reducing agent. Besides, the amounts of N-heterocyclicmethylamine derivatives (V) and amination agents (XII) are each preferably 0.5 to 3.0 times in mole compared with that of the compounds (IV), more preferably 0.8 to 1.5 times in mole. When the amounts of N-heterocyclicmethylamine derivatives (V) and amination agents (XII) are less than 0.5 times in mole, the reaction yield tends to decrease. In addition, when the amounts exceed 3.0 times in mole, the reaction gives a tendency of excess amount of the amination agent. Furthermore, the foregoing reductive amination reaction is preferably conducted in the temperature range of 20 °C or more and 50°C or less. When the foregoing reaction is conducted at the temperature of less than 20°C or more than 50 °C, the reaction yield tends to decrease.

(Alkylation reaction)

Hereafter, an alkylation reaction according to the present invention, that is,

in the foregoing reaction scheme (B), a step for synthesizing N-heterocyclic-alkylamine derivatives (I) from alkylamine derivatives (VI) and heterocyclic metylation agents (VII);

in the foregoing reaction scheme (C), a step for synthesizing N-heterocyclicmethyl-alkylamine derivatives (I-b, tertiary amine) from N-heterocyclicmethyl-alkylamine derivetives (I-a, secondary amine) and alkylation agents having a carbon number of 1 to 5 (XIV);

in the foregoing reaction scheme (D), a step for synthesizing aldehyde derivatives (IV) from aldehyde derivatives of which $R^4$ is an alkyl group having a carbon number of 1 to 5 or a halogenated alkyl group having a carbon number of 1 to 5, and alkylation agents (IX); and

in the foregoing reaction scheme (E), a step for synthesizing aldehyde derivatives (IV) from imine derivatives (X) and alkylation agents (IX);

will be explained.

The alkylation reaction according to the present invention may use a reaction condition of usual alkylation reaction. In addition, this reaction may be conducted in a solvent or in a solvent-free condition.

The used solvents in the alkylation reaction in the solvent are hydrocarbons such as benzene, toluene, xylene and hexane; hydrocarbon halides such as methylenechloride, chloroform and carbon tertrachloride; ethers such as diethylether, diisopropylether, tetrahydrofuran and dioxane; ketones such as acetone and methylethylketone; and others such as acetonitrile, dimethylformamide, 1-methyl-2-pyrrolidinone and dimethylsulfoxide.

Note that the foregoing alkylation reaction is preferably conducted in the presence of a base. When the alkylation reaction is conducted in the presence of the base, the reaction tends to be accelerated. Here, the examples of used based are inorganic bases such as sodium carbonate, potassium carbonate, sodium hydrogencarbonate, sodium hydroxide and potassium hydroxide; alkoxides of alkali metals such as sodium methoxide, sodium ethoxide and potassium t-butoxide; hydrides of alkali metals such as sodium hydride and potassium hydride; organic metal compounds of alkali metals such as lithium diisopropylamide and n-butyllithium; and organic tertiary amines such as triethylamine, pyridine, N,N-dimethylaniline and DBU (1,8-diazabicyclo[5.4.0]undec-7-ene). Moreover, using inorganic bases such as potassium carbonate and sodium carbonate in the steps described by the foregoing reaction schemes (B) and (C); using inorganic bases such as sodium hydroxide and potassium hydroxide in the step described by the foregoing reaction scheme (D); and using organic metal compounds of alkali metals such as lithium diisopropylamide in the step described by the foregoing reaction scheme (E) are particularly preferable because the alkylation reaction tends to be more accelerated. Note that the amount of the above-mentioned bases is preferably 1.0 to 10.0 times in mole compared with each of alkylamine derivatives (VI), N-heterocyclicmethyl-alkylamine derivatives (I-a), aldehyde derivatives (VIII) and imine derivatives (X), and more preferably 1.0 to 2.0 times in mole. When the amount of the base is less than 1.0 times in mole, the reaction yield tends to decrease. On the other hand, when it exceeds 10.0 times in mole, the reaction gives a tendency of excess amount of the base.

Moreover, the amounts of the heterocyclicmethylation agent (VII), the alkylation agents (XIV) and (IX) are each preferably 1.0 to 20.0 times in mole with respect to the compounds (VI), (I-a), (VIII) or (X), and more preferably 1.0 to 4.0 times in mole.

Furthermore, the foregoing alkylation reaction may be conducted in the temperature range from the melting point of the used solvent to the boiling point thereof. However, it is preferable that the reaction described by the foregoing reaction schemes (B) and (C) is conducted at 20 to 100 °C, one described by the foregoing reaction scheme (D) is done at 70 °C to the reflux point, and one described by the foregoing reaction scheme (E) is done at -80 to 20 °C. When the above-described reactions are conducted at the temperature of less than the lower limit value or more than the upper limit value, the reaction yield tends to decrease.

Additionally, the reaction describedby the foregoing reaction scheme (F) may be conducted applying the method written in the following reference:

Tetrahedron Lett., 14, 619(1962).

More specifically, the aldehyde derivatives (IV) may be obtained by reacting the ester derivatives (XI) at a lower temperature with equimolar amount of the reducing agent such as diisobutylaluminium hydride.

(Amide reduction)

The reaction described by the foregoing reaction scheme (H) may be conducted applying the method written in the following reference:

Helv. Chim. Acta., 31, 1397 (1948) ; J. Am. Chem. Soc., 86, 3566(1964).

More specifically, aminederivatives (VI) maybe synthesized by reacting alkylamide derivatives (XIII) with reducing agents such as lithium aluminium hydride and diborane.

(Purification treatment)

After finishing a reaction by the foregoing method, a desired compound (I) may be obtained by carrying out a purification treatment with the synthesized reaction mixture. Here, a conventionally known method can be used for the aforementioned purification treatment. Specifically, at first, the obtained reaction mixture through the foregoing reaction is put in the iced water, then an organic layer is separated by an extraction with organic solvents such as ethylacetate, chloroform, methylenechloride and benzene. Further, after this organic layer is washed with water and dried, the solvent is evaporated under a reduced pressure to get a residue. Thus, the desired compound (I) may be obtained by subjecting the residue to a treatment such as silica gel chromatography.

In the N-heterocyclicmethyl-alkylamine derivatives (I) obtained by the foregoing method, when the second position of a propyl group has an asymmetric carbon, an optical isomer can exist whether or not other substituents have asymmetric points. In the present invention, the compound (I) includes all individual isomers and mixtures which are formed in an arbitrary ratio of each isomers.

(Acid addition salts)

The foregoing N-heterocyclicmethyl-alkylamine derivatives (I) can easily form acid addition salts, therefore, they can be used in the form of inorganic acid salts or organic acid salts thereof. Here, the acids forming acid addition salts are, for example, inorganic acids such as hydrochloric acid, hydrobromic acid, hydriodic acid, nitric acid, sulfuric acid and phosphoric acid; and organic acids such as formic acid, acetic acid, butyric acid, p-toluenesulfonic acid, dodecylbenzenesulfonic acid, camphorsulfonic acid, maleic acid, palmitic acid, stearic acid, oxalic acid, succinic acid, fumaric acid, tartaric acid, citric acid, salicylic acid and saccharin.

(Fungicidal action to phytopathogen)

N-heterocyclicmethyl propylamine derivatives (I) according to the present invention shows preventive effects to plant diseases such as Pyricularia oryzae, Cochliobolus miyabeanus, Xanthomonas oryzae, Rhizoctonia solani, Helminthosporium sigmoiduem, Gibberella fujikuroi, Rythium aphanidermatum, Podosphaera leucotricha, Venturia inaequalis, Monilinia mali, Alternaria alternata, Valsa mali, Alternaria kikuchiana, Phyllactinia pyri, Gymnosporangium asiaticum, Venturia nashicola, Uncinula necator, Plasmopara viticola, Glomerella cingulata, Erysiphe graminis f. sp hordei, Puccinia graminis, Puccinia striiformis, Pyrenophora graminea, Rhynchosporium secalis, Erysiphe graminis f. sp tritici, Puccinia recondita, Puccinia striiformis, Pseudocercosporella herpotrichoides, Microdochium nivale, Leptosphaeria nodorum, Septoria tritici, Sphaerotheca fuliginea, Collectotrichum lagenarium, Pseudoperonospora cubensis, Phytophthora capsici, Erysiphe cichoracearum, Alternaria solani, Erysiphe cichoracearum, Sphaerotheca humuli, Erysiphe cichoracearum, Cercospora beticola, Ustillaga maydis, Monilinia fructicola, Botrytis cinerea which invades various crops and Sclerotinia sclerotiorum. Futher, the aforementioned N-heterocyclicmethyl alkylamine derivatives (I) perform both preventive effect and curative effect to some plant diseases out of the aforementionedplant diseases. Moreover, the aforementioned N-heterocyclicmethyl alkylamine derivatives (I) have a preventive effect to fungous infection in animals including human beings, which are caused by pathogenic fungi such as Candida, Trichophyton, Microsporum, Edpidermophyton, Cryptococcus, Aspergillus, Coccidioides, Paracoccidioides, Histoplasma and Blastomyces.

(Effective ingredient of fungicide)

The fungicide according to the present invention containing the compound (I) as an effective ingredient has an excellent fungicidal activity to many kinds of diseases, and has a sufficiently excellent preventive effect if it is used for applications such as an agricultural chemical to plant diseases and an antifungal agent to fungous infection.

Here, when the compound (I) according to the present invention is used as the effective ingredient of an

agricultural chemical, the compound (I) according to the present invention can be used as an antifungal agent as it stands. However, the compound is used as a formulation for various forms such as dust formulation, water dispersible powder, granule and emulsion together with a formulation coadjuvant which is used if required.

Here, when the fungicide according to the present invention is used as an agricultural chemical, it is preferable that the content of the aforementioned compound (I) is 0.1 to 95 wt% based on the total weight of the fungicide (containing the compound (I) according to the present invention) as the standard (100 wt%), more preferably 0.5 to 90 wt% and further preferably 2 to 70 wt%. When the content of the aforementioned compound (I) is less than 0.1 wt%, a sufficient preventive effect to a plant disease tends to be hardly obtained. When the content exceeds 95 wt%, the content of a formulation coadjuvant to be used is insufficient, as a result, a sufficient effect tends to be hardly obtained as a fungicide.

In addition, taken up as the aforementioned formulation coadjuvant are immobilization carrier, liquid diluent, surfactant, and the like. Here, talc, kaolin, bentonite, diatomearth, white carbon and clay are preferably used as immobilization carrier; and water, xylene, toluene, chlorobenzene, cyclohexane, cyclohexanone, dimethylsulfoxide, dimethylformamide, alcohol and the like are preferably used as liquid diluent. Although it is preferred that surfactant is used depending on the effect, taken up as emulsions are polyoxyethylenealkylaryl ether, polyoxyethylenesorbitanmonolaurate and the like; taken up as dispersants are lignin sulfonate, dibutylnaphthalene sulfonate, and the like, taken up as lubricants are alkyl sulfonate, alkylphenyl sulfonate, and the like and taken up as wetting agents are alkyl sulfonate, alkylphenyl sulfonate, and the like.

Futher, when the fungicide according to the present invention is used as an agricultural chemical, the fungicide according to the present invention may be used as it stands, and may be used by diluting the fungicide at a predetermined concentration with a diluent such as water. Here, when the fungicide is used by dilution with a diluent, it is preferred that the concentration of the compound (I) is 0.001 to 1.0 wt%. In addition, it is preferred that the usage of the compound (I) is 20 to 5,000 g per an agricultural and horticultural land of one hectare such as upland field, paddy field, orchard and green house, and more preferably 50 to 1,000 g. When the usage of the compound (I) is less than the aforementioned lower limit, a sufficient preventive effect to a plant disease tends to be hardly obtained and when the usage exceeds the aforementioned upper limit, the usage of the fungicide tends to be excess.

In addition, since the use concentration and usage of the aforementioned compound (I) in these agricultural chemicals vary with the form of a formulation, use period, use method, use place, object crop or the like, they may be increased or decreased from the aforementioned ranges if required. Moreover, the compound according to the present invention may be used in combination with other effective ingredients, for example, fungicide, insecticide, miticide and herbicide if required.

On the other hand, when the fungicide according to the present invention is used as an antifungal agent, the compound (I) according to the present invention can be used as the antifungal agent as it stands. However, it is usual to use the compound as a mixture with a formulation carrier which is selected corresponding to an administration path and a means of formulation in a specimen. More concretely, the compound is used as an oral administration antifungal agent in the form of a tablet which contains an excipient such as starch or lactose and the compound (I), a capsule which contains only the compound or a mixture of the compound (I) and excipient or ovules, elixir or emulsion which contains flavor or colorant together with the compound (I), or the like. In addition, taken up as non-oral administration antifungal agent is an aseptic aqueous solution which contains salt or glucose adequate to make an aqueous solution and blood isotonic, an administration can be performed to a specimen by giving an intraveous injection or a hypodermic injection of the aforementioned aqueous solution.

In addition, when the fungicide according to the present invention is used as an antifungal agent, the fungicide can be locally used for a specimen in the form of lotion, liquid formulation, cream, ointment or catapasm. Taken up as preferred components contained in the fungicide in the form like this are hydrocarbons such as liquid paraffin, polyhydric alcohol such as polyethyleneglycol, surfactant, stabilizer, antiseptic agent, or the like.

Further, when the fungicide according to the present invention is orally or non-orally administered to a specimen (a human body or the like) as an antifungal agent, it is preferred that the content of the compound in the aforementioned fungicide (the dose per a specimen of 1 kg which is daily administered) is 0.1 to 1 mg/kg based on a daily dose standard. More concretely, when one or not less than two pieces of a tablet or a capsule which contains the compound (I) are administered to a specimen at one time, it is preferred that the content of the compound (I) in the aforementioned tablet or capsule is 5 mg to 0.5 g. In addition, since the dose and content of the compound (I) vary with the age of a specimen, weight, reactivity or the like, they can be increased or decreased from the aforementioned range in accordance with the judgment of a doctor or the like.

## Example

Although the present invention is more concretely described based on the Examples (manufacturing example, formulation example, test example), the present invention isnotlimitedtothebelow-mentioned manufacturing examples,

the formulation examples and the test examples unless the gist of the invention is changed. In addition, the compounds I - 1 to 142 are each the same compounds as those described in the aforementioned Tables 1 to 7.

Manufacturing Example 1

N-[3-(4-t-butylphenyl)-2,2-dimethylpropyl]-6-chlo ro-N-methyl-3-pyridylmethylamine [I-18] was synthesized by the method described by the aforementioned reaction scheme (B).

Sodium hydroxide of 1.0 g was first dissolved in water of 1.0 ml, benzene of 1.5 ml and tetrabutylammonium iodide of 100 mg (0.27 mmol) were added and the solution was heated at 70° C. A solution where isobutylaldehyde of 2.0 g (27.8 mmol) and 4-t-butylbenzylbromide of 4.8 g (21.1 mmol) were dissolved with benzene of 2.0 ml was dropped to the solution and was stirred at 70° C for 8 hours.

After the solution was naturally cooled down, the reaction solution was added to water, and the solution was extracted with benzene. The obtained benzene layer was washed with a saturated saline solution followed by drying with anhydrous sodium sulfate, and the solvent was evaporated under reduced pressure. The residue thus obtained was refined with the silica gel (Silica Gel 60,230-400 mesh, made by Merck Inc.) column [the composition of the eluate: n-Hex (n-hexane)/AcOEt (ethyl acetate) = 30/1], and 3-(4-t-butylphenyl)-2,2-dimethylpropanal (IV-18) of 1.54 g was obtained as a colorless oily matter. The yield was 33.5 %.

Next, sodium cyanoborohydride of 760 mg (12.1 mmol) was suspended in absolute methanol of 40 ml, and after methylamine hydrochloride of 4.0 g (59.3 mmol) was further added thereto, the compound obtained above (IV-18) of 2.60 g (11.9 mmol) /absolute methanol of 10 ml were added little by little. Then, the solution was stirred at a room temperature for 8 hours.

After the reaction was over, methanol was evaporated from the reaction solution under reduced pressure, water was added. Then, after the solution was further controlled at pH 11 with 2N sodium hydroxide solution, the solution was extracted with chloroform. The obtained chloroform layer was washed with a saturated saline solution followed by drying with anhydrous sodium sulfate, and an oily matter of 2.8 gwas obtained by evaporating the solvent under reduced pressure. The oily matter was refined with silica gel (Wakogel C-200, made by Wako Pure Chemical Industries, Ltd) column (the eluate: AcOEt), 3-(4-t-butylphenyl)-N, 2,2-trimethylpropylamine (VI-18) of 1.40 g was obtained as a colorless oily matter. The yield was 50.5 %.

The compound thus obtained (VI-18 ) of 390mg (1.67 mmol) was dissolved in DMF of 2 ml, and 6-chloro-3-chloromethylpyridine of 222 mg (1.37 mmol) was added to the solution. Then, potassium carbonate of 190 mg (1.38 mmol) was further added, and the solution was heated at 50° C for 8 hours.

After the solution was naturally cooled down, the reaction solution was pored into the water, and the solution was extracted with benzene. The obtained benzene layer was washed with a saturated saline solution followed by drying with anhydrous sodium sulfate, an oil matter of 740 mg was obtained by evaporating the solvent under reduced pressure. The oily matter was refined with the silica gel (Silica Gel 60,230-400mesh, made by Merck Inc.) column (the composition of the eluate: n-Hex/AcOEt = 30/1), and the object compound (I-18) was obtained as a colorless oily matter of 510 mg. The yield was 100 %.

Manufacturing Example 2

6-chloro-N-[2,2-dimethyl-3-(4-trifluoromethoxyphe nyl)propyl]-3-pyridylmethylamine (I-45) was synthesized by the method described by the aforementioned reaction scheme (A).

Sodium hydroxide of 0. 4 g was first dissolved in water of 0.4 ml, benzene of 0.4 ml and tetrabutylammonium iodide of 15 mg (0.04 mmol) were added and the solution was heated at 70° C. A solution where isobutylaldehyde of 720 mg (10.0 mmol) and 4-trifluoromethoxybenzylbromide of 1.05 g (4.0 mmol) were dissolved with benzene of 0.4 ml was dropped to the solution and the solution was heated at 70° C for 2 hours.

After the solution was naturally cooled down, the reaction solution was added to water and the solution was extracted with benzene. The obtained benzene layer was washed with a saturated saline solution followed by drying with anhydrous sodium sulfate, the solvent was evaporated under reduced pressure. The residue thus obtained was refined with the silica gel (Silica Gel 60,230-400 mesh, made by Merck Inc.). column [the composition of the eluate: n-Hex (n-hexane)/AcOEt (ethyl acetate) = 20/1], and 2,2-dimethyl-3-(4-trifluoromethoxyphenyl)propanal (IV-45) of 700 mg was obtained as a colorless oily matter. The yield was 71.1 %.

Next, the compound (IV-45) of 600 mg (2.44 mmol) obtained in the aforementioned reaction was dissolved in 1,2-dichloroethane of 12 ml, and then 6-chloro-N-methyl-3-pyridylmethylamine of 458 mg (2.93 mmol) was added. Further, sodium triacetoxyborohydride of 620 mg (2.93 mmol) and acetic acid of 0.14 ml (0.24 mmol) were added, and the solution was stirred at a room temperature for 2 hours.

Thereafter, the reaction solution was poured into a saturated sodium bicarbonate aqueous solution and the solution was extracted with methylene chloride. The obtained methylene chloride layer was washed with a saturated

saline solution followed by drying with anhydrous sodium sulfate, and the solvent was evaporated under reduced pressure. The obtained residue was refined with the silica gel (Silica Gel 60, 230-400 mesh, made by Merck Inc.) column [the composition of the eluate: n-Hex (n-hexane)/AcOEt (ethyl acetate) = 20/1], and the object compound (I-45) of 300 mg was obtained as a light-yellow oily matter. The yield was 31.8 %.

Manufacturing Example 3

N-[3-(4-t-butylcyclohexyl)-2-methylpropyl]-6-chloro-N-methyl-3-pyridylmethylamine (I-62) was synthesized by the method described by the aforementioned reaction scheme (A).

1.5M cyclohexane solution of 4 ml (6.0mmol) of lithium diisopropylamide was first dissolved in anhydrous tetrahydrofuran of 4 ml in the stream of nitrogen and the solution was cooled down at 0° C. After a solution of N-propylidenecyclohexylamine of 828 mg (6.0 mmol)/anhydrous tetrahydrofuran of 3 ml was dropped to the solution, the solution was stirred at 0° C for 15 minutes.

Next, after the reaction solution was cooled down at -75° C, a solution of (4-t-butylcyclohexyl)methylbromide of 700 mg (3.0 mmol) /anhydrous tetrahydrofuran of 3 ml was dropped, the solution was stirred at -78° C for 1 hour, and was further stirred at -20° C for 2 hours. Then, 2N hydrochloric acid solution of 14 ml was added to the reaction solution, after the solution was heated up to a room temperature, the solution was further stirred for 30 minutes.

The reaction solution was neutralized and the solution was extracted with ethyl acetate. The obtained ethyl acetate layer was washed with a saturated saline solution followed by drying with anhydrous sodium sulfate, the solvent was evaporated under reduced pressure. The obtained residue was refined with the silica gel (Wako gel, C-300, made by Wako Pure Chemical Industries, Ltd.) column [the composition of the eluate: n-Hex (n-hexane)/AcOEt (ethyl acetate) = 50/1], and 3-(4-t-butylcyclohexyl)-2-methylpropanal (I-62) of 159 mg was obtained as a light-yellow oily matter. The yield was 25.3 %.

Next, the compound (IV-62) of 159 mg (0.76 mmol) obtained in the aforementioned reaction was dissolved in 1, 2-dichloroethane of 4 ml. 6-chloro-N-methyl-3-pyridylmethylamine of 130 mg (0.83 mmol) was added to the solution, sodium triacetoxyborohydride of 176 mg (0.83mmol) wasfurtheradded and the solution was stirred at a room temperature for 1.5 hours.

Then, the reaction solution was poured into a saturated sodium bicarbonate aqueous solution and the solution was extracted with methylene chloride. The obtained methylene chloride layer was washed with a saturated saline solution followed by drying with anhydrous sodium sulfate, and the solvent was evaporated under reduced pressure.

The obtained residue was refined with the silica gel (Wako gel, C-300, made by Wako Pure Chemical Industries, Ltd.) column [the composition of the eluate: n-Hex (n-hexane)/AcOEt (ethyl acetate) = 15/1], and the object compound (I-62) of 220 mg was obtained as a light-yellow oily matter. The yield was 82.9 %.

Manufacturing Example 4

6-chloro-N-[2-methyl-4-(3-trifluoromethoxyphenyl) butyl]-N-methyl-3-pyridylmethylamine (I-58) was synthesized by the method described by the aforementioned reaction scheme (A).

2-methyl-4-(3-trifluoromethoxyphenyl)methyl butyrate of 591 mg (2.14 mmol) was dissolved in methylene chloride of 9 ml in the atmosphere of nitrogen. After the solution was cooled down at -78° C, 0.95M hexane solution of 2.48 ml (2.35 mmol) of aluminium isobutyl hydride was dropped and the solution was stirred at -78° C for 40 minutes.

After methanol of 5 ml was added to the reaction solution, the solution was heated up to a room temperature and was stirred for 30 minutes. Then, the precipitate was filtered and was taken out, after the precipitate was washed with chloroform, the washing solution and the eluate were mixed. The solvent was evaporated from the organic layer thus obtained under reduced pressure, the residue was refined with the silica gel (Wako gel, C-300, made by Wako Pure Chemical Industries, Ltd.) column [the composition of the eluate: n-Hex (n-hexane)/AcOEt (ethyl acetate) = 30/1], and 2-methyl-4-(3-trifluoromethoxyphenyl)butanal (IV-58) of 405 mg was obtained as a colorless oily matter. The yield was 77.2 %.

Next, the compound (IV-58) obtained in the aforementioned reaction of 385 mg (1.57 mmol) was dissolved in 1,2-dichloroethane of 4 ml, and 6-chloro-N-methyl-3-pyridylmethylamine of 294 mg (1.88 mmol) was added to the solution. Further, sodium triacetoxyborohydride of 497 mg (2.35 mmol) was added thereto and the solution was stirred at a room temperature for 2.5 hours.

After the reaction was over, the reaction solution was poured into the water and the solution was extracted with chloroform. The obtained chloroform layer was washed with a saturated saline solution followed by drying with anhydrous sodium sulfate, and the solvent was evaporated under reduced pressure. The obtained residue was refined with the silica gel (Wako gel, C-300, made by Wako Pure Chemical Industries, Ltd.) column [the composition of the eluate: n-Hex (n-hexane) /AcOEt (ethyl acetate) = 3/1], and the object compound (I-58) of 559 mg was obtained as a colorless oily matter. The yield was 92.3 %.

Manufacturing Example 5

N-[3-(4-t-butylphenyl)-2,2-dimethylpropyl]-N-meth yl-2-pyrazylmethylamine (I-22) was synthesized by the method described by the aforementioned reaction scheme (C).

N-[3-(4-t-butylphenyl)-2,2-dimethylpropyl]-2-pyra zylmethylamine (I-4) of 129 mg (0.73 mmol) was first dissolved in DMF of 2 ml. Methyl iodide of 106 mg (0.75 mmol) was added to the solution, and potassium carbonate of 207 mg (1.50 mmol) was added and the solution was stirred at 60° C for 30 minutes.

After the solution was naturally cooled down, the reaction solution was poured into the water and the solution was extracted with ethyl acetate. The obtained ethyl acetate layer was washed with a saturated saline solution followed by drying with anhydrous sodium sulfate, and the solvent was evaporated under reduced pressure to obtain an oily matter of 144 mg. The oily matter was refined with the silica gel (Silica Gel 60,230-400 mesh, made by Merck Inc.) column [the composition of the eluate: n-Hex (n-hexane)/AcOEt (ethyl acetate) = 1/1], and the object compound (I-22) of 79 mg was obtained as an oily matter. The yield was 56.8 %.

The compounds I-1 to I-17, I-19 to I-21, I-23 to I-44, I-46 to I-57, I-59 to I-61, I-63 to I-85, I-87 to I-89, I-91, I-93 to I-98, I-101 to I-121, I-123 to I-142 shown in Tables 1 to 7 were synthesized by the methods in accordance with the manufacturing examples 1 to 5 mentioned above. Tables 8 to 18 show that the synthesis methods of each compound are based on which of the manufacturing examples 1 to 5. Tables 8 to 18 also show the NMR data of the obtained compounds. In addition, in the NMR measurement of each compound, deuterated chloroform was used as a solvent and tetramethylsilane was used as a reference substance.

In addition, in the below-mentioned formulation examples and test examples, used as the comparison compounds were

6-chloro-N-methyl-N-[2-methyl-3-(2,4-dichlorophenyl)pro pyl]-3-pyridylmethylamine [hereinafter referred to as Compound (a)] represented by the following formula (a):

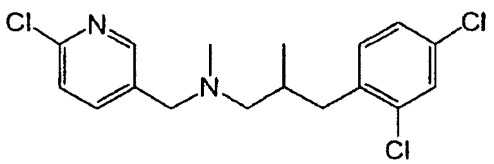

(a)

and

6-chloro-N-methyl-N-[2-methyl-3-(3-chlorophenyl)propyl] -3-pyridylmethylamine [hereinafter referred to as Compound (b)] represented by the following formula (b):

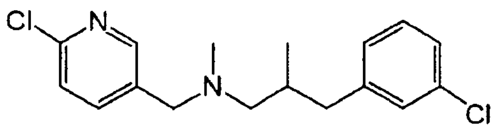

(b)

Table 8

| Compound No. Manufacturing Method[a)] | $^1$H-NMR (δ, ppm) |
|---|---|
| I-1<br>(Manufacturing Example 1) | 0.90 (s, 6H), 1.30 (s, 9H), 2.40 (s, 2H), 2.56 (s, 2H), 3.92 (s, 2H), 7.05 (d, 2H, J=8.2Hz), 7.15~7.18 (m, 1H), 7.24 (d, 2H, J=8.2Hz), 7.35 (d, 1H, J=7.8Hz), 7.63~7.66 (m, 1H), 8.56~8.57 (m, 1H) |
| I-2<br>(Manufacturing Example 1) | 0.88 (s, 6H), 1.30 (s, 9H), 1.44 (s, 1H), 2.33 (s, 2H), 2.52 (s, 2H), 3.78 (s, 2H), 7.02 (d, 2H, J=8.3Hz), 7.24 (d, 2H, J=8.3Hz), 7.29 (d, 1H, J=7.8Hz), 7.67 (dd, 1H, J=2.3Hz, 7.8Hz), 8.34 (d, 1H, J=2.3Hz) |

a) A manufacturing method with a parenthesis ( ) in the Table shows that a compound was manufactured according to a manufacturing example in the parenthesis ( ).

Table 8 (continued)

| Compound No. Manufacturing Method[a)] | $^1$H-NMR (δ, ppm) |
|---|---|
| I-3 (Manufacturing Example 1) | 0.92 (s, 6H), 1.54 (s, 1H), 2.41 (s, 2H), 2.78 (s, 2H), 3.79 (s, 2H), 7.11~7.14 (m, 2H), 7.15~7.19 (m, 1H), 7.29 (d, 1H, J=8.2Hz), 7.33~7.36 (m, 1H), 7.69 (dd, 1H, J=2.3Hz, 8.2Hz), 8.35 (d, 1H, J=2.3Hz) |
| I-4 (Manufacturing Example 1) | 0.93 (s, 6H), 1.30 (s, 9H), 1.72 (s, 1H), 2.42 (s, 2H), 2.57 (s, 2H), 3.98 (s, 2H), 7.06~7.43 (m, 4H), 8.54~8.78 (m, 3H) |
| I-5 (Manufacturing Example 1) | 0.86 (s, 6H), 1.31 (s, 9H), 1.59 (s, 1H), 2.37 (s, 2H), 2.54 (s, 2H), 3.94 (s, 2H), 6.99~7.39 (m, 4H), 8.50~8.58 (m, 2H) |
| I-6 (Manufacturing Example 1) | 0.86 (s, 6H), 1.33 (s, 9H), 2.38 (s, 2H), 2.46 (s, 2H), 3.70 (s, 2H), 3.88 (s, 3H), 7.00~7.46 (m, 6H) |
| I-7 (Manufacturing Example 1) | 0.92 (s, 6H), 1.11 (s, 1H), 2.37 (s, 2H), 2.66 (s, 2H), 3.61 (s, 2H), 3.86 (s, 3H), 6.94 (s, 1H) , 7.06 (d, 2H, J=8.0Hz), 7.13 (d, 2H, J=8.0Hz), 7.26 (s, 1H) |
| I-8 (Manufacturing Example 1) | 0.87 (s, 6H), 0.94 (s, 1H), 2.31 (s, 2H), 2.57 (s, 2H), 3.61 (s, 2H), 3.87 (s, 3H), 6.96 (d, 2H, J=8.2Hz), 7.00 (s, 1H), 7.16 (d, 2H, J=8.2Hz), 7.30 (s, 1H) |
| I-9 (Manufacturing Example 1) | 0.89 (s, 6H), 1.03 (s, 1H), 2.32 (s, 2H), 2.60 (s, 2H), 3.63 (s, 2H), 3.83 (s, 3H), 6.57~7.06 (m, 3H), 7.17 (s, 1H), 7.24 (s, 1H) |
| I-10 (Manufacturing Example 1) | 0.89 (s, 6H), 1.29 (s, 9H), 2.00 (s, 1H), 2.40 (s, 2H), 2.59 (s, 2H), 3.93 (s, 2H), 6.99~7.49 (m, 5H), 8.64~8.77 (m, 1H), 9.14~9.19 (m, 1H) |
| I-11 (Manufacturing Example 1) | 0.83 (s, 6H), 1.29 (s, 9H), 2.46 (s, 2H), 2.56 (s, 2H), 3.87 (s, 2H), 6.73~7.08 (m, 2H), 7.23 (d, 2H, J=8.2Hz), 7.39 (d, 2H, J=8.2Hz), 7.67 (s, 1H) |
| I-12 (Manufacturing Example 1) | 0.83 (s, 6H), 2.34 (s, 2H), 2.56 (s, 2H), 3.76 (s, 2H), 6.54~7.13 (m, 4H), 7.44 (1H,brs), 7.61 (s, 1H) |
| I-13 (Manufacturing Example 1) | 0.88 (s, 6H), 1.31 (s, 9H), 1.53 (s, 1H), 2.39 (s, 2H), 2.54 (s, 2H), 3.78 (s, 2H), 6.02 (d, 1H, J=6.0Hz), 6.14 (dd, 1H, J=2.8Hz, 6.0Hz), 6.74 (d, 1H, J=2.8Hz), 7.04 (d, 2H, J=8.3Hz), 7.29 (d, 2H, J=8.3Hz) |
| I-14 (Manufacturing Example 2) | 0.85 (s, 6H), 1.27 (s, 9H), 2.27 (s, 5H), 2.47 (s, 2H), 3.65 (s, 2H), 6.97 (d, 2H, J=8Hz), 7.22 (d, 2H, J=8Hz), 7.25 (s, 1H) |
| I-15 (Manufacturing Example 2) | 0.83 (s, 6H), 1.27 (s, 9H), 2.25 (s, 3H), 2.32 (s, 2H), 2.43 (s, 2H), 3.67 (s, 2H), 6.90 (d, 2H, J=8Hz), 7.12 (d, 2H, J=8Hz), 6.88~7.32 (m, 1H), 7.42~7.62 (m, 2H), 8.28~8.48 (m, 1H) |

a) A manufacturing method with a parenthesis ( ) in the Table shows that a compound was manufactured according to a manufacturing example in the parenthesis ( ).

Table 9

| Compound No. Manufacturing Method[a)] | $^1$H-NMR (δ, ppm) |
|---|---|
| I-16 (Manufacturing Example 2) | 0.85 (s, 6H), 1.27 (s, 9H), 2.18 (s, 3H), 2.28 (s, 2H), 2.47 (s, 2H), 3.52 (s, 2H), 6.93 (d, 2H, J=8Hz), 7.17 (d, 2H, J=8Hz), 6.73~7.33 (m, 1H), 7.47~7.80 (m, 1H), 8.27~8.57 (m, 2H) |
| I-17 (Manufacturing Example 2) | 0.87 (s, 6H), 1.27 (s, 9H), 2.20 (s, 3H), 2.30 (s, 2H), 2.48 (s, 2H), 3.53 (s, 2H), 6.93 (d, 2H, J=8Hz), 7.17 (d, 2H, J=8Hz), 7.20 (d, 2H, J=6Hz), 8.42 (d, 2H, J=6Hz) |

a) A manufacturing method with a parenthesis ( ) in the Table shows that a compound was manufactured according to a manufacturing example in the parenthesis ( ).

Table 9 (continued)

| Compound No. Manufacturing Method[a)] | $^1$H-NMR (δ, ppm) |
|---|---|
| I-18<br>Manufacturing Example 1 | 0.85 (s, 6H), 1.27 (s, 9H), 2.18 (s, 3H), 2.30 (s, 2H), 2.47 (s, 2H), 3.52 (s, 2H), 6.95 (d, 2H, J=8Hz), 7.20 (d, 2H, J=8Hz), 7.20 (d, 1H, J=8Hz), 7.63 (dd, 1H, J=2, 8Hz), 8.27 (d, 1H, J=2Hz) |
| I-19<br>(Manufacturing Example 2) | 0.85 (s, 6H), 1.28 (s, 9H), 2.18 (s, 3H), 2.28 (s, 2H), 2.45 (s, 2H), 3.50 (s, 2H), 6.77 (dd, 1H, J=3, 8Hz), 6.92 (d, 2H, J=8Hz), 7.18 (d, 2H, J=8Hz), 7.72 (ddd, 1H, J=3, 8, 8Hz), 8.03 (d, 1H, J=3Hz) |
| I-20<br>(Manufacturing Example 1) | 0.85 (s, 6H), 1.28 (s, 9H), 2.27 (s, 3H), 2.38 (s, 2H), 2.47 (s, 2H), 3.65 (s, 2H), 6.8~7.3 (m, 5H), 7.97 (dd, 1H, J=7.2, 1.7Hz), 8.18 (dd, 1H, J=4.4,1.7Hz) |
| I-21<br>(Manufacturing Example 1) | 0.85 (s, 6H), 1.27 (s, 9H), 2.19 (s, 3H), 2.29 (s, 2H), 2.47 (s, 2H), 2.67 (s, 3H), 3.50 (s, 2H), 6.93 (d, 2H, J=8.4Hz), 7.20 (d, 2H, J=8.4Hz), 8.55 (s, 2H) |
| I-22<br>Manufacturing Example 5 | 0.85 (s, 6H), 1.27 (s, 9H), 2.32 (s, 3H), 2.38 (s, 2H), 3.77 (s, 2H), 6.97 (d, 2H, J=8Hz), 7.23 (d, 2H, J=8Hz), 8.38 (bs, 2H), 8.82 (bs, 1H) |
| I-23<br>(Manufacturing Example 1) | 0.87 (s, 6H), 1.28 (s, 9H), 2.43 (s, 7H), 4.93 (s, 2H), 6.92 (d, 2H, J=8Hz), 7.18 (d, 2H, J=8Hz), 7.83 (s, 1H), 7.97 (s, 1H) |
| I-24<br>(Manufacturing Example 1) | 0.75 (s, 6H), 1.28 (s, 9H), 2.20 (s, 3H), 2.35 (s, 2H), 3.40 (s, 2H), 3.60 (s, 3H), 5.90 (d, 2H, J=2.4Hz), 6.47 (dd, 1H, J=2.4, 2.4Hz), 6.93 (d, 2H, J=8.4Hz), 7.20 (d, 2H, J=8.4Hz) |
| I-25<br>(Manufacturing Example 2) | 0.53~1.50 (m, 5H), 0.82 (s, 3H), 1.27 (s, 9H), 2.15 (s, 3H), 2.28 (s, 2H), 2.48 (s, 2H), 3.47 (s, 2H), 6.92 (d, 2H, J=8Hz), 7.13 (d, 1H, J=8Hz), 7.17 (d, 2H, J=8Hz), 7.56 (dd, 1H, J=2, 8Hz), 8.22 (d, 1H, J=2Hz) |
| I-26<br>(Manufacturing Example 4) | 1.27 (s, 9H), 1.57~1.97 (m, 6H), 2.07 (s, 3H), 2.28 (s, 2H), 2.85 (s, 2H), 3.38 (s, 2H), 7.17 (s, 4H), 7.20 (d, 1H, J=8Hz), 7.62 (dd, 1H, J=2, 8Hz), 8.27 (d, 1H, J=2Hz) |
| I-27<br>(Manufacturing Example 2) | 0.87 (s,6H), 1.33 (d, 6H, J=6.3Hz), 2.22 (s, 3H), 2.34 (s, 2H), 2.51 (s, 2H), 3.57 (s, 2H),4.52 (qq, 1H, J=6.3Hz), 6.66~6.76 (m, 3H), 7.12~7.19 (m, 1H), 7.29 (d, 1H, J=8.3Hz), 7.71 (dd, 1H, J=2.0, 8.3Hz), 8.34 (d,1H, J=2.0Hz) |
| I-28<br>(Manufacturing Example 2) | 0.86 (s, 6H), 2.25 (s, 3H), 2.35 (s, 2H), 2.55 (s, 2H), 3.60 (s, 2H), 7.12 (s, 4H), 7.30 (d, 1H, J=8.3Hz), 7.72 (dd, 1H, J=2.4, 8.3Hz), 8.36 (d, 1H, J=2.4Hz) |

a) A manufacturing method with a parenthesis ( ) in the Table shows that a compound was manufactured according to a manufacturing example in the parenthesis ( ).

Table 10

| Compound No. Manufacturing Method[a)] | $^1$H-NMR (δ, ppm) |
|---|---|
| I-29<br>(Manufacturing Example 2) | 0.86 (s, 6H), 2.23 (s, 3H), 2.38 (s, 2H), 2.64 (s, 2H), 3.60 (s, 2H), 7.18~7.30 (m, 5H), 7.75 (dd, 1H, J=2.0, 8.3Hz), 8.32 (d, 1H, J=2.0Hz) |
| I-30<br>(Manufacturing Example 2) | 0.86 (s, 6H), 2.23 (s, 3H), 2.34 (s, 2H), 2.57 (s, 2H), 3.58 (s, 2H), 6.98~7.31 (m, 4H), 7.30 (d, 1H, J=7.3Hz), 7.70 (dd, 1H, J=2.4, 7.3Hz), 8.35 (d, 1H, J=2.4Hz) |

a) A manufacturing method with a parenthesis ( ) in the Table shows that a compound was manufactured according to a manufacturing example in the parenthesis ( ).

Table 10 (continued)

| Compound No. Manufacturing Method[a)] | $^1$H-NMR (δ, ppm) |
|---|---|
| I-31 (Manufacturing Example 2) | 0.86 (s, 6H), 2.25 (s, 3H), 2.35 (s, 2H), 2.55 (s, 2H), 3.60 (s, 2H), 7.12 (s, 4H), 7.30 (d, 1H, J=8.3Hz), 7.72 (dd, 1H, J=2.4, 8.3Hz), 8.36 (d, 1H, J=2.4Hz) |
| I-32 (Manufacturing Example 2) | 0.86 (s, 6H), 2.23 (s, 3H), 2.38 (s, 2H), 2.64 (s, 2H), 3.60 (s, 2H), 7.18~7.30 (m, 5H), 7.75 (dd, 1H, J=2.0, 8.3Hz), 8.32 (d, 1H, J=2.0Hz) |
| I-33 (Manufacturing Example 2) | 0.86 (s, 6H), 2.23 (s, 3H), 2.34 (s, 2H), 2.57 (s, 2H), 3.58 (s, 2H), 6.98~7.31 (m, 4H), 7.30 (d, 1H, J=7.3Hz), 7.70 (dd, 1H, J=2.4, 7.3Hz), 8.35 (d, 1H, J=2.4Hz) |
| I-34 (Manufacturing Example 2) | 0.86 (s, 6H), 2.23 (s, 3H), 2.34 (s, 2H), 2.57 (s, 2H), 3.58 (s, 2H), 6.98~7.31 (m, 4H), 7.30 (d, 1H, J=7.3Hz), 7.70 (dd, 1H, J=2.4, 7.3Hz), 8.35 (d, 1H, J=2.4Hz) |
| I-35 (Manufacturing Example 2) | 0.87 (s, 6H), 2.23 (s, 3H), 2.34 (s, 2H), 2.53 (s, 2H), 3.58 (s, 2H), 6.90~7.11 (m, 7H), 7.27~7.40 (m, 3H), 7.71 (dd, 1H, J=2.4, 8.3Hz), 8.35 (d, 1H, J=2.4Hz) |
| I-36 (Manufacturing Example 2) | 0.85 (s, 6H), 2.18 (s, 3H), 2.30 (s, 2H), 2.50 (s, 2H), 3.52 (s, 2H), 6.67~7.48 (m, 10H), 7.63 (dd, 1H, J=2, 8Hz), 8.28 (d, 1H, J=2Hz) |
| I-37 (Manufacturing Example 2) | 0.92 (s, 6H), 2.19 (s, 3H), 2.38 (s, 2H), 2.62 (s, 2H), 3.56 (s, 2H), 6.85~6.95 (m, 3H), 7.05~7.34 (m, 7H), 7.71 (dd, 1H, J=2.0, 7.8Hz), 8.28 (d, 1H, J=2.0Hz) |
| I-38 (Manufacturing Example 2) | 0.86 (s, 6H), 2.20 (s, 3H), 2.30 (s, 2H), 2.48 (s, 2H), 3.55 (s, 2H), 6.81~7.11 (m, 6H), 7.28~7.34 (m, 3H), 7.67 (dd, 1H, J=2.4, 8.3Hz), 8.32 (d, 1H, J=2.4Hz) |
| I-39 (Manufacturing Example 2) | 0.86 (s, 6H), 2.21 (s, 3H), 2.33 (s, 2H), 2.33 (s, 3H), 2.52 (s, 2H), 3.56 (s, 2H), 6.78~6.92 (m, 5H), 7.10~7.92 (m, 4H), 7.70 (dd, 1H, J=2.4, 8.3Hz), 8.32 (d, 1H, J=2.4Hz) |
| I-40 (Manufacturing Example 2) | 0.90 (s, 6H), 2.22 (s, 3H), 2.33 (s, 2H), 2.54 (s, 2H), 3.57 (s, 2H), 6.80~6.96 (m, 3H), 7.13~7.43 (m, 6H), 7.68 (dd, 1H, J=2.4, 8.3Hz), 8.33 (d, 1H, J=2.4Hz) |
| I-41 (Manufacturing Example 2) | 0.91 (s, 6H), 2.24 (s, 3H), 2.37 (s, 2H), 2.62 (s, 2H), 3.59 (s, 2H), 7.09~7.59 (m, 10H), 7.71 (dd, 1H, J=2.4, 8.3Hz), 8.35 (d, 1H, J=2.4Hz) |
| I-42 (Manufacturing Example 2) | 0.86 (s, 6H), 2.24 (s, 3H), 2.34 (s, 2H), 2.62 (s, 2H), 3.59 (s, 2H), 7.28~7.31 (m, 1H), 7.30 (d, 1H, J=7.8HZ), 7.35~7.38 (m, 2H), 7.41~7.49 (m, 1H), 7.70 (dd, 1H, J=2.4, 8.3Hz), 8.35 (d, 1H, J=2.4Hz) |
| I-43 (Manufacturing Example 2) | 0.85 (s, 6H), 2.25 (s, 3H), 2.34 (s, 2H), 2.57 (s, 2H), 3.59 (s, 2H), 7.10 (t, 1H, J=9.3Hz), 7.24~7.35 (m, 2H), 7.30 (d, 1H, J=7.8Hz), 7.70 (dd, 1H, J=2.4, 7.8Hz), 8.35 (d, 1H, J=2.4Hz) |

a) A manufacturing method with a parenthesis ( ) in the Table shows that a compound was manufactured according to a manufacturing example in the parenthesis ( ).

Table 11

| Compound No. Manufacturing Method[a)] | $^1$H-NMR (δ, ppm) |
|---|---|
| I-44 (Manufacturing Example 2) | 0.90 (s, 6H), 2.26 (s, 3H), 2.41 (s, 2H), 2.87 (s, 2H), 3.62 (s, 2H), 7.30 (d,1H, J=7.8Hz), 7.50 (d, 1H, J=8.3Hz), 7.69~7.74 (m, 2H), 7.90 (s, 1H), 8.35 (d, 1H, J=2.5Hz) |
| I-45 Manufacturing Example 2 | 0.86 (s, 6H), 2.25 (s, 3H), 2.35 (s, 2H), 2.55 (s, 2H), 3.60 (s, 2H), 7.12 (s, 4H), 7.30 (d, 1H, J=8.3Hz), 7.72 (dd, 1H, J=2.4, 8.3Hz), 8.36 (d, 1H, J=2.4Hz) |
| I-46 (Manufacturing Example 2) | 0.86 (s, 6H), 2.23 (s, 3H), 2.34 (s, 2H), 2.57 (s, 2H), 3.58 (s, 2H), 6.98~7.31 (m, 4H), 7.30 (d, 1H, J=7.3Hz), 7.70 (dd, 1H, J=2.4, 7.3Hz), 8.35 (d, 1H, J=2.4Hz) |
| I-47 (Manufacturing Example 2) | 0.86 (s, 6H), 2.23 (s, 3H), 2.38 (s, 2H), 2.64 (s, 2H), 3.60 (s, 2H), 7.18~7.30 (m, 5H), 7.75 (dd, 1H, J=2.0, 8.3Hz), 8.32 (d, 1H, J=2.0Hz) |
| I-48 (Manufacturing Example 2) | 0.86 (s, 6H), 2.23 (s, 3H), 2.34 (s, 2H), 2.56 (s, 2H), 3.58 (s, 2H), 5.91 (tt, 1H, J=2.4, 53.2Hz), 6.98~7.11 (m, 3H), 7.24~7.31 (m, 2H), 7.70 (dd, 1H, J=2.4, 8.3Hz), 8.34 (d, 1H, J=2.4Hz) |
| I-49 (Manufacturing Example 2) | 0.86 (s, 6H), 2.25 (s, 3H), 2.35 (s, 2H), 2.55 (s, 2H), 3.60 (s, 2H), 6.78~6.84 (m, 2H), 6.95 (d, 1H, J=8.3Hz), 7.30 (d, 1H, J=8.3Hz), 7.70 (dd, 1H, J=2.4, 8.3Hz), 8.35 (d, 1H, J=2.4Hz) |
| I-50 (Manufacturing Example 3) | 0.94 (d, 3H, J=6.4Hz), 1.25~1.38 (m, 1H), 1.29 (s, 9H), 1.60~1.80 (m, 2H), 2.05~2.13 (m, 1H), 2.10 (s, 3H), 2.21 (dd, 1H, J=12.2Hz, 6.8Hz), 2.40~2.70 (m, 2H), 3.39 (d, 2H, J=2.4Hz), 7.10 (d, 2H, J=8.8Hz), 7.26 (d, 1H, J=7.8Hz), 7.29 (d, 2H, J=8.8Hz), 7.62 (dd, 1H, J=2.4Hz, 8.8Hz), 8.27 (d, 1H, J=2.4Hz) |
| I-51 (Manufacturing Example 3) | 0.95 (s, 6H), 1.30 (s, 9H), 1.50~1.60 (m, 2H), 2.21 (s, 3H), 2.32 (s, 2H), 2.45~2.60 (m, 2H), 3.56 (s, 2H), 7.10 (d, 2H, J=8.3Hz), 7.26 (d, 2H, J=8.3Hz), 7.30 (d, 2H, J=8.3Hz), 7.69 (dd, 1H, J=2.0Hz, J=8.3Hz), 8.32 (d, 1H, J=2.0Hz) |
| I-52 (Manufacturing Example 3) | 0.87 (d, 3H, J=6.8Hz), 1.30 (s, 9H), 1.29~1.70 (m, 5H), 2.13 (s, 3H), 2.00~2.15 (m, 2H), 2.50~2.60 (m, 2H), 3.40 (d, 2H, J=5.4Hz), 7.10 (d, 2H, J=8.3Hz), 7.25 (d, 1H, J=8.3Hz), 7.30 (d, 2H, J=8.3Hz), 7.62 (dd, 1H, J=2.4Hz, 8.3Hz), 8.27 (d, 1H, J=2.4Hz) |
| I-53 (Manufacturing Example 3) | 0.85 (s, 6H), 1.25~1.35 (m, 2H), 1.29 (s, 9H), 1.45~1.60 (m, 2H), 2.15 (s, 3H), 2.23 (s, 2H), 2.51 (t, 2H, J=7.3Hz), 3.49 (s, 2H), 7.10 (d, 2H, J=8.3Hz), 7.24 (d, 1H, J=2.4Hz), 7.28 (d, 2H, J=8.3Hz), 7.65 (dd, 1H, J=2.0Hz, 8.3Hz), 8.29 (d, 1H, J=2.0Hz) |
| I-54 (Manufacturing Example 3) | 0.91 (d, 3H, J=6.4Hz), 1.20~1.40 (m, 1H), 1.50~1.80 (m, 2H), 2.09 (s, 3H), 2.00~2.25 (m, 2H), 2.40~2.70 (m, 2H), 3.39 (s, 2H), 6.75~7.10 (m, 6H), 7.15~7.35 (m, 4H), 7.60 (dd, 1H, J=2.4Hz, J=8.3Hz), 8.25 (d, 1H, J=2.4Hz) |
| I-55 (Manufacturing Example 3) | 0.93 (s, 6H), 1.49~1.58 (m, 2H), 2.19 (s, 3H), 2.30 (s, 2H), 2.49~2.56 (m, 2H), 3.54 (s, 2H), 6.75~7.19 (m, 6H), 7.20~7.35 (m, 4H), 7.66 (dd, 1H, J=2.0Hz, J=8.3Hz), 8.29 (d, 1H, J=2.0Hz) |

a) A manufacturing method with a parenthesis ( ) in the Table shows that a compound was manufactured according to a manufacturing example in the parenthesis ( ).

Table 12

| Compound No. Manufacturing Method[a)] | $^1$H-NMR (δ, ppm) |
|---|---|
| I-56<br>(Manufacturing Example 4) | 0.97 (d, 3H, J=6.8Hz), 1.23~1.38 (m, 1H), 1.65~1.81 (m, 2H), 2.12 (s, 3H), 2.17~2.28 (m, 2H), 2.60~2.81 (m, 2H), 3.42 (s, 2H), 7.10~7.18 (m, 2H), 7.26 (d, 1H, J=8.3Hz), 7.34 (d, 1H, J=1.5Hz), 7.62 (dd, 1H, J=2.4, 8.3Hz,), 8.28 (d, 1H, J=2.4Hz) |
| I-57<br>(Manufacturing Example 4) | 0.92 (d, 3H, J=6.4Hz), 1.18~1.35 (m, 1H), 1.50~1.70 (m, 2H), 2.00~2.23 (m, 2H), 2.11 (s, 3H), 2.40~2.70 (m, 2H), 3.41 (s, 2H), 7.04 (d, 2H, J=1.5Hz), 7.16 (m, 1H, J=1.5Hz), 7.27 (d, 1H, J=8.8Hz), 7.61 (dd, 1H, J=2.4Hz, 8.8Hz), 8.27 (d, 1H, J=2.4Hz) |
| I-58<br>Manufacturing Example 4 | 0.93 (d, 3H, J=6.8Hz), 1.28~1.41 (m, 1H), 1.63~1.83 (m, 2H), 2.04~2.28 (m, 2H), 2.10 (s, 3H), 2.50~2.76 (m, 2H), 3.40 (s, 2H), 7.01~7.11 (m, 3H), 7.24~7.31 (m, 2H), 7.61 (dd, 1H, J=2.5, 8.3Hz), 8.27 (d, 1H, J=2.5Hz) |
| I-59<br>(Manufacturing Example 3) | 0.94 (s, 6H), 1.49~1.56 (m, 2H), 2.21 (s, 3H), 2.31 (s, 2H), 2.53~2.59 (m, 2H), 3.55 (s, 2H), 7.00~7.08 (m, 3H), 7.25 (d, 1H, J=8.3Hz), 7.25~7.30 (m, 1H), 7.67 (dd, 1H, J=2.4, 8.3Hz), 8.30 (d, 1H, J=2.4Hz) |
| I-60<br>(Manufacturing Example 4) | 0.93 (d, 3H, J=6.3Hz), 1.24~1.40 (m, 1H), 1.61~1.82 (m, 2H), 2.06~2.24 (m, 2H), 2.15 (s, 3H), 2.50~2.73 (m, 2H), 3.40 (s, 2H), 7.10 (d, 2H, J=8.8Hz), 7.17 (d, 2H, J=8.8Hz), 7.26 (d, 1H, J=8.3Hz), 7.60 (dd, 1H, J=2.5, 8.3Hz), 8.28 (d, 1H, J=2.5Hz) |
| I-61<br>(Manufacturing Example 3) | 0.95 (s, 6H), 1.50~1.56 (m, 2H), 2.22 (s, 3H), 2.31 (s, 2H), 2.52~2.59 (m, 2H), 3.56 (s, 2H), 7.11 (d, 2H, J=8.8Hz), 7.16 (d, 2H, J=8.8Hz), 7.26 (d, 1H, J=8.3Hz), 7.67 (dd, 1H, J=2.4, 8.3Hz), 8.32 (d, 1H, J=2.4Hz) |
| I-62<br>Manufacturing Example 3 | 0.83 (s, 9H), 0.85 (d, 3H, J=6.4Hz), 0.70~1.25 (m, 8H), 2.07 (d, 2H, J=7.8Hz), 1.60~1.82 (m, 5H), 2.13 (s, 3H), 3.40 (d, 1H, J=13.7Hz), 3.44 (d, 1H, J=13.7Hz), 7.28 (d, 1H, J=8.3Hz), 7.65 (dd, 1H, J=8.3Hz, J=2.4Hz), 8.29 (d, 1H, J=2.0Hz) |
| I-63<br>(Manufacturing Example 3) | 0.81 (s, 3H), 0.87 (s, 6H), 0.88~1.25 (m, 8H), 1.65~1.80 (m, 4H), 2.13 (s, 3H), 2.22 (s, 2H), 3.53 (s, 2H), 7.27 (d, 1H, J=8.3Hz), 7.69 (dd, 1H, J=8.3Hz, J=2.4Hz), 8.31 (d, 1H, J=2.4Hz) |
| I-64<br>(Manufacturing Example 3) | 0.70~1.25 (m, 11H), 0.82 (s, 9H), 1.30~1.45 (m, 2H), 1.50~1.80 (m, 5H), 1.95~2.20 (m, 2H), 2.12 (s, 3H), 3.38 (d, 1H, J=13.8Hz) , 3.45 (d, 1H, J=13.8Hz), 7.26 (d, 1H, J=8.3Hz) , 7.64 (dd, 1H, J=8.3Hz, J=2.4Hz), 8.28 (d, 1H, J=2.4Hz) |
| I-65<br>(Manufacturing Example 3) | 0.70~1.25 (m, 9H), 0.81 (s, 9H), 0.82 (s, 6H), 1.35~1.58 (m, 2H), 1.65~1.78 (m, 3H), 2.17 (s, 3H), 2.22(s, 2H), 3.52(s, 2H), 7.26 (d, 1H, J=8.3Hz), 7.68 (dd, 1H, J=8.3Hz, J=2.4Hz), 8.31 (d, 1H, J=2.0Hz) |
| I-66<br>(Manufacturing Example 3) | 0.80~1.38 (m, 12H), 0.82 (s, 9H), 0.86 (d, 3H, J=6.8Hz), 1.50~1.80 (m, 5H), 2.00~2.20 (m, 2H), 2.13 (s, 3H), 3.39 (d, 1H, J=13.7Hz) , 3.43 (d, 1H, J=13.7Hz), 7.27 (d, 1H, J=8.3Hz), 7.64 (dd, 1H, J=8.3Hz, J=2.4Hz), 8.28 (d, 1H, J=2.4Hz) |
| I-67<br>(Manufacturing Example 3) | 0.70~1.25 (m, 12H), 0.82 (s, 9H), 0.84 (s, 6H), 1.60~1.80 (m, 4H), 2.17 (s, 3H), 2.22 (s, 2H), 3.52 (s, 2H), 7.27 (d, 1H, J=7.8Hz), 7.69 (dd, 1H, J=7.8Hz, J=2.4Hz), 8.30 (d, 1H, J=2.0Hz) |

a) A manufacturing method with a parenthesis ( ) in the Table shows that a compound was manufactured according to a manufacturing example in the parenthesis ( ).

Table 13

| Compound No. Manufacturing Method[a)] | $^1$H-NMR (δ, ppm) |
|---|---|
| I-68 (Manufacturing Example 2) | 0.84 (s, 6H), 2.21 (s, 3H), 2.30 (s, 2H), 2.47 (s, 2H), 3.56 (s, 2H), 3.79 (s, 3H), 6.80 (d, 2H, J=8.8Hz), 7.02 (d, 2H, J=8.8Hz), 7.28 (d, 1H, J=8.3Hz), 7.70 (dd, 1H, J=2.4, 8.3Hz), 8.33 (d, 1H, J=2.4Hz) |
| I-69 (Manufacturing Example 2) | 0.86 (s, 6H), 2.21 (s, 3H), 2.32 (s, 2H), 2.51 (s, 2H), 3.56 (s, 2H), 3.79 (s, 3H), 6.66~6.77 (m, 3H), 7.14~7.21 (m, 1H), 7.28 (d, 1H, J=8.3Hz), 7.70 (dd, 1H, J=2.4, 8.3Hz), 8.34 (d, 1H, J=2.0Hz) |
| I-70 (Manufacturing Example 2) | 0.84 (s, 6H), 2.21 (s, 3H), 2.35 (s, 3H), 2.59 (s, 2H), 3.57 (s, 2H), 3.78 (s, 3H), 6.82~6.89 (m, 2H), 7.05~ 7.09 (m, 1H), 7.14~7.21 (m, 1H), 7.27 (d, 1H, J=8.3Hz), 7.73 (dd, 1H, J=2.4, 8.4Hz),8.34 (d, 1H, J=2.0Hz) |
| I-71 (Manufacturing Example 2) | 0.23~0.73 (m, 4H), 0.85 (s, 6H), 2.20 (s, 3H), 2.30 (s, 2H), 2.47 (s, 2H), 3.53 (s, 2H), 3.75 (d, 2H, J=7Hz), 6.78 (d, 2H, J=8Hz), 7.03 (d, 2H, J=8Hz), 7.28 (d, 1H, J=8Hz), 7.72 (dd, 1H, J=2, 8Hz), 8.37 (d, 1H, J=2Hz) |
| I-72 (Manufacturing Example 2) | 0.30~0.39 (m, 2H), 0.58~0.68 (m, 2H), 0.86 (s, 6H), 1.18~1.30 (m, 1H), 2.21 (s, 3H), 2.32 (s, 2H), 2.50 (s, 2H), 3.77 (d, 2H, J=6.8Hz), 6.60~6.76 (m, 3H), 7.15 (m, 1H), 7.28 (d, 1H, J=8.3Hz), 7.70 (dd, 1H, J=2.4, 8.3Hz), 8.33 (d, 1H, J=2.4Hz) |
| I-73 (Manufacturing Example 2) | 0.85 (s, 6H), 1.33 (s, 9H), 2.25 (s, 3H), 2.35 (s, 2H), 2.50 (s, 2H), 3.60 (s, 2H), 6.88 (d, 2H, J=8.4Hz), 7.00 (d, 2H, J=8.4Hz), 7.29 (d, 1H, J=8.2Hz), 7.74 (dd, 1H, J=2.3, 8.2Hz),8.34 (d, 1H, J=2.0Hz) |
| I-74 (Manufacturing Example 2) | 0.85 (s, 6H), 1.13 (d, 6H, J=6.4Hz), 2.21 (s, 3H), 2.32 (s, 2H), 2.47 (s, 2H), 3.57 (s, 2H), 4.47~4.57 (m, 1H), 6.79 (d, 2H, J=8. 8Hz) , 7.02 (d, 2H, J=8.8Hz), 7.29 (d, 1H, J=8.3Hz), 7.72 (dd, 1H, J=2.0, 8.3Hz), 8.35 (d, 1H, J=2.4Hz) |
| I-75 (Manufacturing Example 2) | 0.83 (s, 6H), 1.00 (dx2, 6H), 1.82~2.12 (m, 1H), 2.20 (s, 3H), 2.30 (s, 2H), 2.45 (s, 2H), 3.53 (s, 2H), 3.67 (d, 2H, J=6Hz), 6.77 (d, 2H, J=8Hz), 7.02 (d, 2H, J=8Hz), 7.27 (d, 1H, J=8Hz), 7.70 (dd, 1H, J=2, 8Hz), 8.35 (d, 1H, J=2Hz) |
| I-76 (Manufacturing Example 2) | 0.68~1.98 (m, 5H), 0.83 (s, 6H), 1.25 (d, 3H, J=6Hz), 2.18 (s, 3H), 2.28 (s, 2H), 2.45 (s, 2H), 3.52 (s, 2H), 4.02~4.42 (m, 1H), 6.73 (d, 2H, J=8Hz), 6.98 (d, 2H, J=8Hz), 7.23 (d, 1H, J=8Hz), 7.67 (dd, 1H, J=2, 8Hz), 8.32 (d, 1H, J=2Hz) |
| I-77 (Manufacturing Example 2) | 0.83 (s, 6H), 2.18 (s, 3H), 2.28 (s, 2H), 2.45 (s, 2H), 3.53 (s, 2H), 4.35~4.62 (m, 2H), 5.08~5.58 (m, 2H), 5.75~6.52 (m, 1H), 6.82 (d, 2H, J=8Hz), 7.05 (d, 2H, J=8Hz), 7.28 (d, 1H, J=8Hz), 7.73 (dd, 1H, J=2, 8Hz), 8.40 (d, 1H, J=2Hz) |
| I-78 (Manufacturing Example 2) | 0.85 (s, 6H), 2.22 (s, 3H), 2.32 (s, 2H), 2.48 (bs, 3H), 3.55 (s, 2H), 4.65 (d, 2H, J=3Hz), 6.90 (d, 2H, J=8Hz), 7.10 (d, 2H, J=8Hz), 7.30 (d, 1H, J=8Hz), 7.75 (dd, 1H, J=2, 8Hz), 8.40 (d, 1H, J=2Hz) |
| I-79 (Manufacturing Example 2) | 0.88 (s, 6H), 1.26~1.58 (m, 6H), 1.60~1.86 (m, 2H), 1.95~2.19 (m, 2H), 2.23 (s, 3H), 2.34 (s, 2H), 2.50 (s, 2H), 3.58 (s, 2H), 4.18~4.25 (m, 1H), 6.67~6.85 (m, 3H), 7.12~7.19 (m, 1H), 7.29 (d, 1H, J=8.8Hz), 7.70~7.75 (m, 1H), 8.34 (d, 1H, J=2.4Hz) |

a) A manufacturing method with a parenthesis ( ) in the Table shows that a compound was manufactured according to a manufacturing example in the parenthesis ( ).

Table 14

| Compound No. Manufacturing Method[a)] | $^1$H-NMR (δ, ppm) |
|---|---|
| I-80 (Manufacturing Example 2) | 0.85 (s, 6H), 2.21 (s, 3H), 2.32 (s, 2H), 2.51 (s, 2H), 3.56 (s, 2H), 5.05 (s, 2H), 6.72~6.86 (m, 3H), 7.15~7.45 (m, 7H), 7.67 (dd, 1H, J=2.0, 8.3Hz), 8.34 (d, 1H, J=2.0Hz) |
| I-81 (Manufacturing Example 2) | 0.13 (s, 9H), 0.83 (s, 6H), 2.20 (s, 3H), 2.30 (s, 2H), 2.47 (s, 2H), 3.55 (s, 2H + s, 2H), 6.87 (d, 2H, J=8Hz), 7.08 (d, 2H, J=8Hz), 7.32 (d, 1H, J=8Hz), 7.77 (dd, 1H, J=2, 8Hz), 8.42 (d, 1H, J=2Hz) |
| I-82 (Manufacturing Example 2) | 0.83 (s, 6H), 2.22 (s, 3H), 2.30 (s, 2H), 2.48 (s, 2H), 3.55 (s, 2H), 4.30 ( q, 2H, J=8Hz), 6.83 ( q, 2H, J=8Hz), 7.08 (d, 2H, J=8Hz), 7.27 (d, 1H, J=8Hz), 7.70 (dd, 1H, J=2, 8Hz), 8.37 (d, 1H, J=2Hz) |
| I-83 (Manufacturing Example 2) | 0.86 (s, 6H), 2.23 (s, 3H), 2.34 (s, 2H), 2.54 (s, 2H), 3.58 (s, 2H), 5.91 (tt, 1H, J=2.9, 53.2Hz), 7.12 (s, 4H), 7.30 (d, 1H, J-8.3Hz), 7.71 (dd, 1H, J=2.0, 8.3Hz), 8.36 (d, 1H, J=2.0Hz) |
| I-84 (Manufacturing Example 2) | 0.83 (s, 6H), 2.20 (s, 3H), 2.30 (s, 2H), 2.47 (s, 2H), 3.53 (s, 2H), 4.30 (t, 2H, J=12Hz), 6.03 ( tt, 1H, J=4, 54Hz), 6.80 (d, 2H, J=8Hz), 7.07 (d, 2H, J=8Hz), 7.27 (d, 1H, J=8Hz), 7.70 (dd, 1H, J=2, 8Hz), 8.35 (d, 1H, J=2Hz) |
| I-85 (Manufacturing Example 2) | 0.83 (s, 6H), 2.22 (s, 3H), 2.30 (s, 2H), 2.48 (s, 2H), 3.55 (s, 2H), 4.32 (t, 2H, J=12Hz), 6.83 (d, 2H, J=8Hz), 7.10 (d, 2H, J=8Hz), 7.28 (d, 1H, J=8Hz), 7.72 (dd, 1H, J=2, 8Hz), 8.38 (d, 1H, J=2Hz) |
| I-87 (Manufacturing Example 2) | 0.85 (s, 6H), 1.85 (bs, 1H), 2.22 (s, 3H), 2.31 (s, 2H), 2.53 (s, 2H), 3.54 (s, 2H), 4.62 (s, 2H), 7.10 (d,2H, J=8.0Hz), 7.26 (d, 2H, J=8.0Hz), 7.26 (d, 1H, J=8.0Hz), 7.66 (dd, 1H, J=2.4, 8.2Hz), 8.35 (d, 1H, J=2.0Hz) |
| I-88 (Manufacturing Example 2) | 0.86 (s, 6H), 2.21 (s, 3H), 2.32 (s, 2H), 2.54 (s, 2H), 3.39 (s, 3H), 3.56 (s, 2H), 4.42 (s, 2H), 7.09 (d, 2H, J=8.1Hz), 7.22 (d, 2H, J=8.1Hz), 7.27 (d, 1H, J=8.1Hz), 7.68 (dd, 1H, J=2.4, 8.1Hz), 8.33 (d, 1H, J=2.0Hz) |
| I-89 (Manufacturing Example 2) | 0.86 (s, 6H), 1.24 (t, 3H, J=7.0Hz), ), 2.21 (s, 3H), 2.32 (s, 2H), 2.53 (s, 2H), 3.54 (q, 2H, J=7.0Hz), 3.56 (s, 2H), 4.47 (s, 2H), 7.08 (d, 2H, J=7.9Hz), 7.23 (d, 2H, J=7.9Hz), 7.27 (d, 1H, J=8.2Hz),7.68 (dd, 1H, J=2.4, 8.2Hz), 8.33 (d, 1H, J=2.0Hz) |
| I-91 (Manufacturing Example 2) | 0.19~0.22 (m, 2H), 0.51~0.56 (m, 2H), 0.85 (s, 6H), 1.09 (m, 1H), 2.23 (s, 3H), 2.32 (s, 2H), 2.53 (s, 2H), 3.36 (d, 2H, J=6.9Hz), 3.36 (s, 2H), 4.50 (s, 2H), 7.08 (d, 2H, J=8.0Hz), 7.23 (d, 2H, J=8.0Hz), 7.27 (d, 1H, J=8.2Hz), 7.69 (dd, 1H, J=2.4, 8.2Hz), 8.33 (d, 1H, J=2.0Hz) |
| I-93 (Manufacturing Example 2) | 0.85 (s, 6H), 2.22 (s, 3H), 2.32 (s, 2H), 2.55 (s, 2H), 3.57 (s, 2H), 4.12 ( sept, 1H, J=6Hz), 7.17 (d, 2H, J=8Hz), 7.30 (d, 1H, J=8Hz), 7.32 (d, 2H, J=8Hz), 7.73 (dd, 1H, J=2, 8Hz), 8.40 (d, 1H, J=2Hz) |

a) A manufacturing method with a parenthesis ( ) in the Table shows that a compound was manufactured according to a manufacturing example in the parenthesis ( ).

Table 15

| Compound No. Manufacturing Method[a)] | $^1$H-NMR (δ, ppm) |
|---|---|
| I-94 (Manufacturing Example 2) | 0.84 (s, 6H), 1.47 (d, 3H, J=6.6Hz), 2.21 (s, 3H), 2.30 (s, 2H), 2.51 (s, 2H), 3.52 (s, 2H), 4.67~5.11 (m, 1H), 7.00~7.44 (m, 5H), 7.71 (dd, 1H, J=2.4, 8.4Hz), 8.27 (d, 1H, J=2.4Hz) |
| I-95 (Manufacturing Example 2) | 0.86 (s, 6H), 1.42 (d, 3H, J=6.5Hz), 2.23 (s, 3H), 2.33 (s, 2H), 2.53 (s, 3H), 3.22 (s, 2H), 3.57 (s, 2H), 4.28 (q, 1H, J=6.5Hz), 7.10~7.47 (m, 5H), 7.77 (dd, 1H, J=2.4, 8.3Hz), 8.43 (d, 1H, J=2.0Hz) |
| I-96 (Manufacturing Example 2) | 0.87 (s, 6H), 1.17 (t, 3H, J=7.0Hz), 1.42 (d, 3H, J=6.6Hz), 2.23 (s, 3H), 2.34 (s, 2H), 2.53 (s, 2H), 3.36 (q, 2H, J=7.0Hz), 3.58 (s, 2H), 4.40 (q, 1H, J=6.6Hz), 7.00~7.43 (m, 5H), 7.77 (dd, 1H, J=2.4, 8.0Hz), 8.43 (d, 1H, J=2.4Hz) |
| I-97 (Manufacturing Example 2) | 0.12 (m, 2H), 0.50 (m, 2H), 0.86 (s, 6H), 1.04 (m, 1H), 1.45 (d, 3H, J=6.5Hz), 2.23 (s, 3H), 2.34 (s, 2H), 2.53 (s, 2H), 3.08 (dd, 1H, J=7.0, 10.1Hz), 3.18 (dd, 1H, J=6.7, 10.1Hz), 3.58 (s, 2H), 4.40 (q, 1H, J=6.5Hz), 7.08 (d, 2H, J=8.0Hz), 7.20 (d, 2H, J=8.0Hz), 7.28 (d, 1H, J=8.1Hz), 7.70 (dd, 1H, J=2.4, 8.1Hz), 8.34 (d, 1H, J=2.4Hz) |
| I-98 (Manufacturing Example 2) | 0.85 (s, 6H), 1.58 (s, 6H), 1.89 (bs, 1H), 2.22 (s, 3H), 2.31 (s, 2H), 2.52 (s, 2H), 3.55 (s, 2H), 7.08 (d, 2H, J=8.3Hz), 7.27 (d, 1H, J=8.3Hz), 7.37 (d, 2H, , J=8.3Hz), 7.69 (dd, 1H, J=2.4, 8.3Hz),8.26 (d, 1H, J=2.4Hz) |
| I-101 (Manufacturing Example 2) | 0.85 (s, 3H), 0.86 (s, 3H), 1.58 (d, 3H, J=6.4Hz), 2.23 (s, 3H), 2.33 (s, 2H), 2.55 (s, 2H), 3.57 (s, 2H), 3.98 (sept., 1H, J=6.0Hz), 4.80 (q, 1H, J=6.4Hz), 7.13 (d, 2H, J=8.0Hz), 7.21 (d, 2H, J=8.0Hz), 7.28 (d, 1H, J=8.2Hz), 7.68 (dd, 1H, J=2.4, 8.2Hz), 8.33 (d, 1H, J=2.0Hz) |
| I-202 (Manufacturing Example 2) | 0.86 (s, 6H), 2.23 (s, 3H), 2.35 (s, 2H), 2.63 (s, 2H), 3.58 (s, 2H), 7.27 (d, 2H, J=8.1Hz), 7.27 (d, 2H, J=8.1Hz), 7.67 (dd, 1H, J=2.4, 8.2Hz), 7.77 (d, 2H, J=8.1Hz), 8.35 (d, 1H, J=2.0Hz), 9.98 (s, 1H) |
| I-103 (Manufacturing Example 2) | 0.86 (s, 6H), 2.23 (s, 3H), 2.35 (s, 2H), 2.60 (s, 3H), 2.61 (s, 2H), 3.58 (s, 2H), 7.21 (d, 2H, J=7.8Hz), 7.29 (d, 2H, J=7.8Hz), 7.70 (dd, 1H, J=2.4, 8.3Hz), 7.86 (d, 2H, J=8.3Hz), 8.36 (d, 1H, J=2.0Hz) |
| I-104 (Manufacturing Example 2) | 0.87 (s, 6H), 2.23 (s, 3H), 2.34 (s, 2H), 2.58 (s, 3H), 2.60 (s, 2H), 3.57 (s, 2H), 7.22~7.59 (m, 3H), 7.65~7.97 (m, 3H), 8.40 (d, 1H, J=2.4Hz) |
| I-105 (Manufacturing Example 2) | 0.86 (s, 6H), 1.20 (t, 3H, J=7.4Hz), 2.23 (s, 3H), 2.34 (s, 2H), 2.60 (s, 2H), 2.98 (q, 2H, J=7.4Hz), 3.57 (s, 2H), 7.23 (d, 2H, , J=8.0Hz), 7.38 (m, 1H), 7.74 (dd, 1H, J=2.4, 8.4Hz),8.08 (d, 2H, J=8.0Hz), 8.40 (d, 2H, J=2.4Hz) |

a) A manufacturing method with a parenthesis ( ) in the Table shows that a compound was manufactured according to a manufacturing example in the parenthesis ( ).

Table 16

| Compound No. Manufacturing Method[a)] | $^1$H-NMR (δ, ppm) |
|---|---|
| I-106 (Manufacturing Example 2) | 0.86 (s, 6H), 1.21 (d, 6H, J=6.8Hz), 2.24 (s, 3H), 2.35 (s, 2H), 2.61 (s, 2H), 3.57 (sept., 1H, J=7.0Hz), 3.58 (s, 2H), 7.27~7.40 (m, 3H), 7.68~7.73 (m, 2H), 7.80 (dd, 1H, J=2.0, 7.0Hz), 8.35 (d, 1H, J=2.0Hz) |

a) A manufacturing method with a parenthesis ( ) in the Table shows that a compoundwas manufactured according to a manufacturing example in the parenthesis ( ).

Table 16 (continued)

| Compound No. Manufacturing Method[a)] | $^1$H-NMR (δ, ppm) |
|---|---|
| I-107<br>(Manufacturing Example 2) | 0.85 (s, 6H), 2.19 (s, 3H), 2.20 (s, 3H), 2.33 (s, 2H), 2.60 (s, 2H), 3.57 (s, 2H), 6.16 (s, 1H), 7.20 (d, 2H, , J=8.3Hz), 7.29 (d, 1H, J=8.3Hz), 7.69 (dd, 1H, J=2.4, 8.3Hz), 7.78 (d, 2H, J=8.3Hz), 8.35 (d, 1H, J=2.4Hz) |
| I-108<br>(Manufacturing Example 2) | 0.87 (s, 6H), 2.21 (s, 3H), 2.33 (s, 2H), 2.61 (s, 2H), 3.56 (s, 2H), 7.30～7.81 (m, 11H), 8.32 (d, 1H, J=1.5Hz) |
| I-109<br>(Manufacturing Example 2) | 0.86 (s, 6H), 1.66 (s, 3H), 2.22 (s, 3H), 2.33 (s, 2H), 2.53 (s, 2H), 3.57 (s, 2H), 3.75～3.85 (m, 2H), 4.00～4.10 (m, 2H), 7.09 (d, 2H, J=7.8Hz) , 7.29 (d, 1H, J=8.3Hz) , 7.36 (d, 2H, J=7.8Hz), 7.71 (dd, 1H, J=2.4, 8.3Hz), 8.34 (d, 1H, J=2.4Hz) |
| I-110<br>(Manufacturing Example 2) | 0.85 (s, 6H), 1.64 (s, 3H), 2.23 (s, 3H), 2.34 (s, 2H), 2.57 (s, 2H), 3.58 (s, 2H), 3.70～3.88 (m, 2H), 3.90～4.11 (m, 2H), 7.00～7.44 (m, 5H) , 7.80 (dd, 1H, J=2.4, 9.0Hz), 8.34 (d, 1H, J=2.4Hz) |
| I-111<br>(Manufacturing Example 2) | 0.87 (s, 6H + s, 6H), 2.20 (s, 2H), 2.23 (s, 2H), 2.28 (s, 3H + s, 3H), 2.32 (s, 2H), 2.35 (s, 2H), 2.54 (s, 2H), 2.57 (s, 2H), 3.55 (s, 2H + s, 2H), 3.78 (s, 3H + s, 3H), 6.09 (s, 1H), 6.30 (s, 1H), 7.30～7.41 (m, 5H + m,5H), 7.57～7.87 (m, 1H + m, 1H), 8.33～8.43 (m, 1H + m,1H) |
| I-112<br>(Manufacturing Example 2) | 0.88 (s, 6H), 2.23 (s, 3H), 2.35 (s, 2H), 2.65 (s, 3H), 3.58 (s, 2H), 7.24 (d, 2H, J=8.6Hz), 7.29 (d, 1H, J=8.6Hz), 7.71 (dd, 1H, J=2.4, 8.0Hz), 7.96 (d, 2H, J=8.0Hz), 8.35 (d, 1H, J=2.4Hz) |
| I-113<br>(Manufacturing Example 2) | 0.88 (s, 6H), 2.24 (s, 3H), 2.36 (s, 2H), 2.63 (s, 2H), 3.59 (s, 2H), 7.28 (d, 2H, J=8.5Hz), 7.30 (d, 1H, J=8.0Hz), 7.71 (dd, 1H, J=2.4, 8.5Hz), 8.01 (d, 2H, J=8.5Hz), 8.36 (d, 1H, J=2.4Hz) |
| I-114<br>(Manufacturing Example 2) | 0.85 (s, 6H), 2.04 (s, 1H), 2.23 (s, 3H), 2.33 (s, 2H), 2.56 (s, 2H), 3.57 (s, 2H), 4.89 (bs, 2H), 7.15 (d, 2H, J=8.3Hz), 7.29 (d, 1H, J=8.3Hz), 7.52 (d, 2H, J=8.3Hz), 7.71 (dd, 1H,J=2.4, 8.3Hz), 8.32 (d, 1H, J=2.4Hz) |
| I-115<br>(Manufacturing Example 2) | 0.86 (s, 6H), 1.50～1.85 (bs, 1H), 2.22 (s, 3H), 2.28 (s, 3H), 2.34 (s, 2H),2.56 (s, 2H), 3.58 (s, 2H), 7.13 (d, 2H, J=8.3Hz), 7.29 (d, 1H, J=8.3Hz), 7.53 (d, 2H, J=8.3Hz), 7.71 (dd, 1H, J=2.4, 8.3Hz), 8.35 (d, 1H, J=2.4Hz) |
| I-116<br>(Manufacturing Example 2) | 0.86 (s, 6H), 2.22 (s, 6H), 2.33 (s, 2H), 2.55 (s, 2H), 3.57 (s, 2H), 3.99 (s, 3H), 7.12 (d, 2H, J=8.3Hz), 7.29 (d, 1H, J=8.3Hz), 7.54 (d, 2H, J=8.3Hz), 7.70 (dd, 1H, J=2.4, 8.3Hz), 8.35 (d, 1H, J=2.4Hz) |
| I-117<br>(Manufacturing Example 2) | 0.86 (s, 6H), 1.33 (t, 3H, J=6.8Hz), 2.21 (s, 3H), 2.23 (s, 3H), 2.33 (s, 2H), 2.55 (s, 2H), 3.57 (s, 2H), 4.24 (q, 2H, J=6.8Hz), 7.12 (d, 2H, J=8.3Hz), 7.29 (d, 1H, J=8.3Hz), 7.55 (d, 2H, J=8.3Hz), 7.71 (dd, 1H, J=2.4, 8.3Hz), 8.35 (d, 1H, J=2.4Hz) |
| I-118<br>(Manufacturing Example 2) | 0.86 (s, 6H), 1.33 (t, 3H, J=6.8Hz), 2.21 (s, 3H), 2.23 (s, 3H), 2.33 (s, 2H), 2.55 (s, 2H), 3.57 (s, 2H), 4.24 (q, 2H, J=6.8Hz), 7.12 (d, 2H, J=8.3Hz), 7.29 (d, 1H, J=8.3Hz), 7.55 (d, 2H, J=8.3Hz), 7.71 (dd, 1H, J=2.4, 8.3Hz), 8.35 (d, 1H, J=2.4Hz) |

a) A manufacturing method with a parenthesis ( ) in the Table shows that a compoundwas manufactured according to a manufacturing example in the parenthesis ( ).

Table 17

| Compound No. Manufacturing Method[a)] | $^1$H-NMR (δ, ppm) |
|---|---|
| I-119<br>(Manufacturing Example 2) | 0.25~0.35 (m, 2H), 0.48~0.60 (m, 2H), 0.86 (s, 6H), 1.18~1.25 (m, 1H), 2.21 (s, 3H), 2.25 (s, 3H), 2.33 (s, 2H), 2.55 (s, 2H), 3.57 (s, 2H), 4.00 (d, 2H, J=6.8Hz), 7.11 (d, 2H, J=8.3Hz), 7.29 (d, 1H, J=8.3Hz),7.55 (d, 2H, J=8.3Hz), 7.70 (dd, 1H, J=2.4, 8.3Hz), 8.35 (d, 1H, J=2.0Hz) |
| I-120<br>(Manufacturing Example 2) | 0.85 (s, 6H), 2.22 (s, 3H), 2.27 (s, 3H), 2.33 (s, 2H), 2.56 (s, 2H), 3.57 (s, 2H), 4.54 (q, 2H, J=6.8Hz), 7.13 (d, 2H, J=8.0Hz), 7.29 (d, 1H, J=8.0Hz), 7.54 (d, 2H, J=8.5Hz), 7.70 (dd, 1H, J=2.4, 8.5Hz), 8.35 (d, 1H, J=2.4Hz) |
| I-121<br>(Manufacturing Example 2) | 0.86 (s, 6H), 1.11 (t, 3H, J=7.4Hz), 2.21 (s, 3H), 2.32 (s, 2H), 2.54 (s, 2H), 2.73 (q, 2H, J=7.4Hz), 3.55 (s, 2H), 3.95 (s, 3H), 7.13 (d, 2H, , J=8.0Hz), 7.29 (d, 1H, J=8.0Hz), 7.58~7.86 (m, 3H), 8.38 (d, 2H, , J=2.4Hz) |
| I-123<br>(E+Z)<br>(Manufacturing Example 2) | 0.84, 0.85 (s×2, 6H), 1.09, 1.14 (d×2, 6H, J=6.7Hz), 2.21 (s, 3H), 2.32 (s, 2H), 2.55 (s, 2H), 2.78, 3.49 (sept.×2, 1H, J=6.7Hz), 3.56 (s, 2H), 3.76, 3.92 (s×2, 3H), 6.90~7.10 (m, 3H), 7.16~7.26 (m, 1H), 7.28 (d, 1H, J=8.6Hz), 7.68~7.73 (m, 1H),8.33 (d, 1H, J=2.4Hz) |
| I-124<br>(isomer 1)<br>(Manufacturing Example 2) | 0.86 (s, 6H), 2.22 (s, 3H), 2.33 (s, 2H), 2.57 (s, 2H), 3.96 (s, 3H), 7.13 (d, 1H, J=7.3Hz), 7.23~7.44 (m, 4H), 7.71 (dd, 1H, J=2.4, 8.3Hz),8.04 (s, 1H), 8.35 (d, 1H, J=2.4Hz) |
| I-125<br>(isomer 2)<br>(Manufacturing Example 2) | 0.86 (s, 6H), 2.22 (s, 3H), 2.33 (s, 2H), 2.58 (s, 2H), 4.00 (s, 3H), 7.16 (d, 1H, J=7.3Hz), 7.20~7.34 (m, 4H), 7.57 (s, 1H), 7.70 (dd, 1H, J=2.4, 8.3Hz), 7.76 (d, 1H, J=7.8Hz), 8.35 (d, 1H, J=2.0Hz) |
| I-126<br>(Manufacturing Example 2) | 0.86 (s, 6H), 2.20 (s, 3H), 2.21 (s, 3H), 2.32 (s, 2H), 2.55 (s, 2H), 3.57 (s, 2H), 3.96 (s, 3H), 7.10~7.50 (m, 5H), 7.72 (dd, 1H, J=2.4, 8.3Hz), 8.42 (d, 1H, J=2.4Hz) |
| I-127<br>(Manufacturing Example 2) | 0.87 (s, 6H), 1.30 (t, 3H, J=7.4Hz), 2.21 (s, 6H), 2.33 (s, 2H), 2.57 (s, 2H), 3.57 (s, 2H), 4.21 (q, 2H, J=7.4Hz), 7.10~7.62 (m, 5H) , 7.76 (dd, 1H, J=2.4, 8.4Hz), 8.40 (d, 1H, J=2.4Hz) |
| I-128<br>(Manufacturing Example 2) | 0.87 (s, 6H), 1.27 (d, 3H, J=6.4Hz), 2.19 (s, 3H), 2.21 (s, 3H), 2.32 (s, 2H) , 2.57 (s, 2H) , 3.57 (s, 2H) , 4.42 (sept., 1H, J=6.0Hz), 7.10~7.62 (m, 5H) , 7.73 (dd, 1H, J=2.4, 8.4Hz), 8.39 (d, 1H, J=2.4Hz) |
| I-129<br>(E+Z)<br>(Manufacturing Example 2) | 0.85, 0.87 (s×2, 6H), 1.10, 1.17 (d×2, 6H, J=6.7Hz), 2.21, 2.23 (s×2, 3H), 2.32, 2.34 (s×2, 2H), 2.54 (s, 2H), 2.79, 3.48 (sept.×2, 1H, J=7.3Hz), 3.56, 3.57 (s×2, 2H), 3.79, 3.94 (s×2, 3H), 7.10 (d, 2H, J=7.9Hz), 7.29 (d, 1H, J=7.9Hz), 7.30 (d, 2H, J=7.9Hz), 7.70 (dd, 1H, J=2.4, 7.9Hz), 8.34 (d, 1H, J=2.4Hz) |
| I-130<br>(Manufacturing Example 2) | 0.85 (s, 6H), 2.24 (s, 3H), 2.35 (s, 2H), 2.61 (s, 2H), 3.59 (s, 2H), 7.23 (d, 2H, J=8.3Hz), 7.30 (d, 1H, J=8.3Hz), 7.57 (d, 2H, J=8.3Hz), 7.69 (dd, 1H, J=2.4, 8.3Hz), 8.36 (d, 1H, J=2.4Hz) |

a) A manufacturing method with a parenthesis ( ) in the Table shows that a compound was manufactured according to a manufacturing example in the parenthesis ( ).
b) The NMR data of the compounds I-123 and I-129 in the Table are of the mixture of two kinds of geometrical isomers (E isomer and Z isomer) to imino group.

Table 18

| Compound No. Manufacturing Method[a)] | $^1$H-NMR (δ, ppm) |
|---|---|
| I-131 (Manufacturing Example 2) | 0.84 (s, 6H), 2.24 (s, 3H), 2.34 (s, 2H), 2.58 (s, 2H), 3.58 (s, 2H), 7.29~7.38 (m, 2H), 7.41 (s, 1H), 7.49~7.53 (m, 1H), 7.69 (dd, 1H, J=2.0, 8.0Hz), 8.35 (d, 1H, J=2.0Hz) |
| I-132 (Manufacturing Example 2) | 0.86 (s, 6H), 1.58 (s, 9H), 2.22 (s, 3H), 2.34 (s, 2H), 2.60 (s, 2H), 3.58 (s, 2H), 7.26~7.31 (m, 3H), 7.60~7.76 (m, 2H), 7.82 (dd, 1H, J=2.0, 6.8Hz), 8.34 (d, 1H, J=2.0Hz) |
| I-133 (Manufacturing Example 2) | 0.83 (s, 6H), 2.19 (s, 3H), 2.29 (s, 2H), 2.51 (s, 2H), 3.54 (s, 2H), 3.96 (s, 2H), 6.90~7.05 (m, 3H), 7.10~7.20 (m, 4H), 7.20~7.29 (m, 4H) , 7.68 (dd, 1H, J=2. 4, 8. 3Hz) , 8.33 (d, 1H, J=2.4Hz) |
| I-134 (Manufacturing Example 2) | 0.68 (s, 6H), 2.24 (s, 3H), 2.35 (s, 2H), 2.61 (s, 2H), 3.59 (s, 2H), 7.23 (d, 2H, J=7.8Hz), 7.30 (d, 1H, J=8.3Hz), 7.52 (d, 2H, J=8.3Hz), 7.70 (dd, 1H, J=2.4, 8.3Hz), 8.36 (d, 1H, J=2.4Hz) |
| I-135 (Manufacturing Example 2) | 0.87 (s, 6H), 2.23 (s, 3H), 2.34 (s, 2H), 2.54 (s, 2H), 3.58 (s, 2H), 5.20 (d, 1HJ=10.9Hz), 5.71 (d, 1H, J=17.6Hz), 6.70 (dd, 1H, J=10.9, 17.6Hz), 7.08 (d, 2H, J=8.0Hz), 7.28 (d, 2H, J=8.2Hz), 7.28 (d, 1H, J=8.2Hz), 7.31 (d, 2H, J=8.0Hz), 7.70 (dd, 1H, J=2.0, 8.2Hz), 8.35 (d, 1H, J=2.0Hz) |
| I-136 (Manufacturing Example 2) | 0.86 (s, 6H), 2.14 (dd, 3H, J=0.7, 1.4Hz), 2.22 (s, 3H), 2.33 (s, 2H), 2.53 (s, 2H), 3.57 (s, 2H), 5.04 (m, 2H), 5.35 (m, 1H), 7.07 (d, 2H, J=8.3Hz), 7.27 (d, 2H, J=8.2Hz), 7.36 (d, 1H, J=8.3Hz), 7.69 (dd, 1H, J=2.4, 8.1Hz), 8.34 (d, 1H, J=2.0Hz) |
| I-137 (Manufacturing Example 1) | 0.90 (s, 3H), 2.22 (s, 3H), 2.37 (d, 1H, J=14Hz), 2.43 (d, 1H, J=14Hz), 2.70 (d, 1H, J=14Hz), 2.80 (d, 1H, J=14Hz), 3.27 (d, 1H, J=11Hz), 3.53 (d, 1H, J=11Hz), 3.63 (s, 2H), 7.18 (s, 4H), 7.27 (d, 1H, J=8Hz), 7.67 (dd, 1H, J=2, 8Hz), 8.37 (d, 1H, J=2Hz) |
| I-138 (Manufacturing Example 2) | 0.85 (s, 6H), 2.23 (s, 3H), 2.33 (s, 2H), 2.55 (s, 2H), 3.60 (s, 2H), 7.10 (d, 1H, J=9.2Hz), 7.14 (d, 1H, J=9.2Hz), 7.25 (dd, 1H, J=4.0, 8.0Hz), 7.70 (dd, 1H, J=1.8, 8.0Hz), 8.50 (dd, 1H, J=1.8, 4.8Hz), 8.59 (d, 1H, J=1.8Hz) |
| I-139 (Manufacturing Example 2) | 0.85 (s, 6H), 2.23 (s, 3H), 2.33 (s, 2H), 2.55 (s, 2H), 3.56 (s, 2H), 7.12 (s, 4H), 7.45 (d, 1H, J=8.0Hz), 7.60 (dd, 1H, J=2,4, 8.5Hz), 8.34 (d, 1H, J=1.8Hz) |
| I-140 (Manufacturing Example 2) | 0.85 (s, 6H), 2.23 (s, 3H), 2.33 (s, 2H), 2.55 (s, 2H), 3.58 (s, 2H), 6.90 (dd, 1H, J=3.0, 8.5Hz), 7.12 (s, 4H), 7.83 (dd, 1H, d, J=2.4, 8.5Hz),8.16 (m, 1H) |
| I-141 (Manufacturing Example 2) | 0.87 (s, 6H), 2.25 (s, 3H), 2.37 (s, 2H), 2.57 (s, 2H), 3.70 (s, 2H), 7.12 (s, 4H), 7.65 (d, 1H, J=8.6Hz), 7.89 (dd, 1H, J=1.8, 8.5Hz), 8.74 (m, 1H) |
| I-142 (Manufacturing Example 2) | 0.85 (s, 6H), 2.30 (s, 3H), 2.32 (s, 2H), 2.55 (s, 2H), 3.72 (s, 2H), 7.13 (s, 4H), 7.33 (s, 1H) |

a) A manufacturing method with a parenthesis ( ) in the Table shows that a compound was manufactured according to a manufacturing example in the parenthesis ( ).

Formulation Example 1

(Formulation of powder material)

Each component described below was crushed and mixed to prepare the powder material.

| | (Parts by weight) |
|---|---|
| Compound (I-18) | 3 |
| Clay | 40 |
| Talc | 57 |

Formulation Example 2

(Formulation of wettable powder)

Each component described below was crushed and mixed to prepare the wettable powder.

| | (Parts by weight) |
|---|---|
| Compound (I-45) | 50 |
| Lignin sulfonate | 5 |
| Alkyl sulfonate | 3 |
| Diatom earth | 42 |

Formulation Example 3

(Formulation of granule)

Each component described below was uniformly mixed, and water was added, then the material was kneaded, the kneaded material was further granulated by an extruding-type granulating machine and was dried to prepare the granule.

| | (Parts by weight) |
|---|---|
| Compound (I-58) | 5 |
| Bentonite | 43 |
| Clay | 45 |
| Lignin sulfonate | 7 |

Formulation Example 4

(Formulation of emulsion)

Each component described below was uniformly mixed and melted to prepare the emulsion.

| | (Parts by weight) |
|---|---|
| Compound (I-62) | 20 |
| Polyoxyethylenealkylallylether | 10 |
| Polyoxyethylenesorbitanmonolaurate | 3 |
| Xylene | 67 |

Four forms of medicines (powder material, wettable powder, granule and emulsion) of each compound were obtained by using the compounds I-1 to 142 and the comparison compounds (a) and (b) according to the present invention in the same methods as those in the formulation examples 1 to 4.

The tests described below were conducted by use of the medicines obtained in the aforementioned methods.

Test Example 1

(Preventive effect test of Erysiphe graminis f. sp striiformis)

The wettable powders obtained by using each compound were diluted with water and were suspended at a predetermined concentration (250 mg/l) and the solution was sprayed over the first to second leaf stage-wheat (variety: Agriculture and Forestry No. 64) cultivated by using a square plastic pot (size: 6.4 cm x 6.4 cm) at a rate of 100 1/10 a(are).

After the sprayed leaves were air-dried, the suspension of the spore of Erysiphe graminis f. sp tritici germ sampled from the contracted leaves were sprayed and inoculated over the sprayed leaf after air-dried, the leaves were kept at 20 to 24° C under a high-humidity condition for 24 hours, then, the leaves were subjected to still standing in a greenhouse (temperature: 20 to 24° C and relative humidity: 20 to 70 RH). The morbidity rate was investigated based on the following investigation standard on 9th to 14th day after the inoculation and the preventive value was calculated according to the following formula 1.

(Investigation Standard)

| <Morbidity Rate> | <Severity> |
|---|---|
| 0 | Leaf which was not contracted |
| 0.5 | Leaf whose lesion area rate was less than 1 % |
| 1 | Leaf whose lesion area rate was less than 5 % |
| 2 | Leaf whose lesion area rate was 5 % or higher and less than 10 % |
| 3 | Leaf whose lesion area rate was 10 % or higher and less than 30 % |
| 4 | Leaf whose lesion area rate was 30 % or higher and less than 50 % |
| 5 | Leaf whose lesion area rate was 50 % or higher |

(Formula 1)

Preventive value = (1 - morbidity rate of treated area/morbidity rate of untreated area) x 100 %

The preventive values of each compound thus obtained are shown in the following Table 19.

Table 19

| Compound | Preventive Value (%) |
|---|---|
| I-2 | 100 |
| I-18 | 100 |
| I-19 | 100 |
| I-25 | 100 |
| I-27 | 100 |
| I-28 | 100 |
| I-45 | 100 |
| I-49 | 100 |
| I-56 | 100 |
| I-57 | 100 |
| I-58 | 100 |

Table 19 (continued)

| Compound | Preventive Value (%) |
|---|---|
| I-60 | 100 |
| I-62 | 100 |
| I-73 | 100 |
| I-74 | 100 |
| I-82 | 100 |
| I-83 | 100 |
| I-84 | 100 |
| I-85 | 100 |
| I-88 | 100 |
| I-89 | 100 |
| I-95 | 100 |
| I-96 | 100 |
| I-116 | 100 |
| I-117 | 100 |
| I-121 | 100 |
| I-139 | 100 |
| I-140 | 100 |
| I-141 | 100 |
| I-142 | 100 |
| (a) | 60 |
| (b) | 70 |

Test Example 2

(Preventive effect test of cucumber gray mold)

The wettable powders obtained by using each compound were diluted with water and were suspended at a predetermined concentration (250 mg/l) and the solution was sprayed over the first to second leaf stage-cucumber (variety: "Sagamihanpakufushinari") cultivated by using a square plastic pot (size: 6.4 cm x 6.4 cm) at a rate of 100 1/10 a.

After the sprayed leaves were air-dried, a paper disk where the solution of the spore of the gray mold suspended with water was impregnated was put on the aforementioned sprayed leaves, and the leaves were contracted in a glass greenhouse (temperature: 20 to 24° C and relative humidity: 20 to 70 RH). The morbidity rate was investigated based on the following investigation standard on 4th to 6th day after the inoculation and the preventive value was calculated according to the following formula 2.

(Investigation Standard)

| <Morbidity Rate> | <Severity> |
|---|---|
| 0 | Leaf which was not contracted |
| 0.5 | Leaf whose lesion area rate was less than 1 % |
| 1 | Leaf whose lesion area rate was less than 5 % |

(continued)

| <Morbidity Rate> | <Severity> |
|---|---|
| 2 | Leaf whose lesion area rate was 5 % or higher and less than 10 % |
| 3 | Leaf whose lesion area rate was 10 % or higher and less than 30 % |
| 4 | Leaf whose lesion area rate was 30 % or higher and less than 50 % |
| 5 | Leaf whose lesion area rate was 50 % or higher |

(Formula 2)

Preventive value = (1 - morbidity rate of treated area/morbidity rate of untreated area) x 100 %

The preventive values of each compound thus obtained are shown in the following Table 20.

Table 20

| Compound | Preventive Value (%) |
|---|---|
| I-16 | 100 |
| I-18 | 100 |
| I-19 | 100 |
| I-20 | 100 |
| I-21 | 100 |
| I-25 | 100 |
| I-28 | 100 |
| I-29 | 100 |
| I-31 | 100 |
| I-32 | 100 |
| I-33 | 100 |
| I-34 | 100 |
| I-35 | 100 |
| I-36 | 100 |
| I-38 | 100 |
| I-39 | 100 |
| I-40 | 100 |
| I-45 | 100 |
| I-46 | 100 |
| I-48 | 100 |
| I-50 | 100 |
| I-51 | 100 |
| I-52 | 100 |
| I-54 | 100 |
| I-60 | 100 |
| I-62 | 100 |

Table 20 (continued)

| Compound | Preventive Value (%) |
|---|---|
| I-63 | 100 |
| I-64 | 100 |
| I-66 | 100 |
| I-73 | 100 |
| I-74 | 100 |
| I-82 | 100 |
| I-83 | 100 |
| I-84 | 100 |
| I-85 | 100 |
| I-88 | 100 |
| I-89 | 100 |
| I-95 | 100 |
| I-96 | 100 |
| I-116 | 100 |
| I-117 | 100 |
| I-121 | 100 |
| I-139 | 100 |
| I-140 | 100 |
| I-141 | 100 |
| I-142 | 100 |
| (a) | 80 |
| (b) | 30 |

Test Example 3

(Preventive effect of cucumber powdery mildew)

The wettable powders obtained by using each compound were diluted with water and were suspended at a predetermined concentration (100 mg/l) and the solution was sprayed over the first to second leaf stage-cucumber (variety: "Sagamihanpakufushinari") cultivated by using a square plastic pot (size: 6.4 cm x 6.4 cm) at a rate of 100-1/10 a.

After the sprayed leaves were air-dried, the spore was inoculated over the aforementioned air-dried leaves from the contracted leaves with a brush, and the contraction was performed in a glass greenhouse (temperature: 20 to 24° C and relative humidity: 20 to 70 RH). The morbidity rate was investigated based on the same investigation standard as that in the preventive effect test of the cucumber gray mold on 9th to 14th day after the inoculation, and the preventive value was calculated according to the aforementioned formula 2.

The preventive values obtained on each compound are shown in the following Table 21.

Table 21

| Compound | Preventive Value (%) |
|---|---|
| I-19 | 100 |
| I-45 | 100 |
| I-49 | 100 |

Table 21 (continued)

| Compound | Preventive Value (%) |
|---|---|
| I-55 | 100 |
| I-56 | 100 |
| I-57 | 100 |
| I-73 | 100 |
| I-74 | 100 |
| I-82 | 100 |
| I-83 | 100 |
| I-84 | 100 |
| I-85 | 100 |
| I-88 | 100 |
| I-89 | 100 |
| I-95 | 100 |
| I-96 | 100 |
| I-116 | 100 |
| I-117 | 100 |
| I-121 | 100 |
| I-139 | 100 |
| I-140 | 100 |
| I-141 | 100 |
| I-142 | 100 |
| (a) | 70 |
| (b) | 80 |

Test Example 4

(Antibacterial test to various phytopathogen)

In the test examples, a test was performed on the antibacterial of the compounds according to the present invention to various phytopathogenic molds in the methods later described.

<Test Methods>

The compounds of 10 mg were each weighed and they were each dissolved in dimethylsulfoxide of 1 ml. The solution of 0.6 ml was added to PDA culture medium (potato-dextrose-agar culture medium) of 60 ml at about 60° C, the solutions were well mixed in an Erlenmeyer flask of 100 ml, and the solution was poured into a Schale and was solidified therein to prepare a plate culture containing the compound according to the present invention of final concentration of 100 mg/l.

On the other hand, the sample molds on the plate cultured beforehand were punched by a cork borer of diameter of 4 mm, and they were inoculated on the plate culture containing the aforementioned chemical material. After the inoculation, the molds were inoculated at the appropriate growth temperatures of each mold (for the appropriate growth temperatures, for example, the Reference, LIST OF CULTURE 1996 MICROORGANISM 10th edition published by Institute for Fermentation can be referred to.) for 1 to 3 days, the growth rates of the molds were evaluated by measuring the diameter of a colony. The growth rate of the mold thus obtained on the plate culture containing the chemical material is compared with the growth rate of the mold in the non-addition area of the chemical material to find the suppression rate of the mold elongation by the following formula 3.

(Formula 3)

$$R = 100 (dc-dt)/dc$$

[wherein, R is the suppression rate of the mold elongation (%), dc is the diameter of a colony on an untreated plate and dt is the diameter of a colony on a chemical-treated plate, respectively.]

The results obtained above were evaluated in 5-steps in accordance with the following standard.

<Inhibition rate of growth>

5: Mold whose suppression rate of the mold elongation is 90 % or higher
4: Mold whose suppression rate of the mold elongation is less than 90 % and 70 % or higher
3: Mold whose suppression rate of the mold elongation is less than 70 % and 40 % or higher
2: Mold whose suppression rate of the mold elongation is less than 40 % and 20 % or higher
1: Mold whose suppression rate of the mold elongation is less than 20 %

The obtained evaluation results are shown in the following Table 22. In addition, the meanings of the abbreviated letters in Table 22 are as follows:

B. c. ; Botrytis cinerea
P. h. ; Pseudocercosporella herpotrichoides
M. n. ; Microdohium nivale
L. n. ; Leptosphaeria nodorum
V. i. : Venturia inaequalis

Table 22

| Compound | Sample Bacteria | | | | |
|---|---|---|---|---|---|
| | B.c. | P.h. | M.n. | L.n. | V.i. |
| I-18 | 5 | 5 | 5 | 5 | 5 |
| I-45 | 5 | 5 | 5 | 5 | 5 |
| I-57 | 5 | 5 | 5 | 5 | 5 |
| I-58 | 5 | 5 | 5 | 5 | 5 |
| I-60 | 5 | 5 | 5 | 5 | 5 |

Test Example 5

(Antibacterial test to Candida albicans)

In the test examples, a test was performed on the antibacterial of the compounds according to the present invention to Candida albicans in the methods later described.

<Test Method>

The culture dilution train containing each compound was prepared by using a 96-hole flat-bottomed plate, and the inoculation mold solution controlled at 1 to 5 x $10^3$ cell/ml beforehand of 10 μl was added to each hole on the plate. After the inoculation, the molds were cultivated at 35° C for 72 hours, and the propagation of the mold was evaluated by measuring the absorbance at 540 nm. With such a test, the propagation of the mold in this case was compared with the propagation of the mold in the chemical-free test area to find the concentration of a compound where the propagation rate of the mold is suppressed by 80 % as IC 80 (80 % growth inhibition concentration).

The obtained evaluation results are shown in the following Table 23.

Table 23

| Compound | 80% Growth Inhibition Concentration (IC80) [μg/ml] |
|---|---|
| I-4 | 75 |
| I-6 | 18 |
| I-10 | 75 |
| I-11 | 150 |
| I-12 | 18 |

Test Example 6

(Antibacterial test to Aspergillus fumigatus)

In the test example, a test was performed on the antibacterial of the compounds according to the present invention to Aspergillus fumigatus in the method later described.

<Test Method>

Aspergillus fumigatus was cultivated in Sabouraud's agar at 37° C for 3 days, after the spores to be used for the test were formed, the spore suspension was prepared. The spore suspension was suspended in YNB culture to which a 0.22 % low melting point-agarose was added so as to allow the final concentration tobe 1 x $10^4$ cell/ml, and the solution was poured into each hole on a 96-hole flat-bottomed plate. Next, the dilution trains using each of the compounds according to the present invention were added, the solution was cultivated at 25° C for 72 hours, and the propagation rate of the mold was evaluated by measuring the absorbance at 620 nm. With such a test, the propagation rate of the mold in the chemical-free test area was compared with this case to find the concentration of the compound where the propagation rate of the mold is suppressed by 80 % as IC 80 (80 % growth inhibition concentration).

The obtained evaluation results are shown in the following Table 24.

Table 24

| Compound | 80% Growth Inhibition Concentration (IC80) [μg/ml] |
|---|---|
| I-1 | 5 |
| I-2 | 38 |
| I-3 | 9 |
| I-4 | 75 |
| I-5 | 9 |
| I-6 | 9 |
| I-7 | 150 |
| I-9 | 150 |
| I-10 | 38 |
| I-13 | 18 |

As shown in Tables 19 to 24, it is confirmed that a high preventive effect to various plant diseases and a high antibacterial to various disease germs are obtained by using the compounds according to the present invention.

**Industrial Applicability**

As described above, the present invention provides a newly prepared N-hetrocyclicmethyl-alkylamine derivatives, a manufacturing method of the same and fungicides containing the same as an effective ingredient. They are suitably used for agricultural and horticultural fungicides and medicinal antifungal agents, and have a high sterilization

activity and low toxicity to men and beasts, and are of high safety in handling.

## Claims

1. A N-heterocyclicmethyl-alkylamine derivative represented by the following general formula (I) or an acid addition salt thereof:

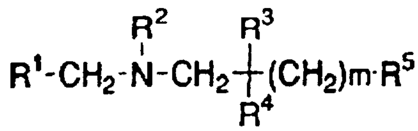

(I)

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

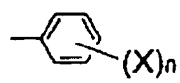

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

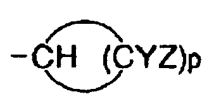

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different), when m is 1 and $R^4$ is a hydrogen atom, $R^5$ is a cycloalkyl group described by the foregoing formula (III).]

2. A method of manufacturing a N-heterocyclicmethyl-alkylamine derivative including a process which obtains a N-heterocyclicmethyl-alkylamine derivative represented by the following formula (I):

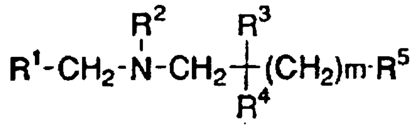

( I )

from an aldehyde derivative represented by the following formula (IV):

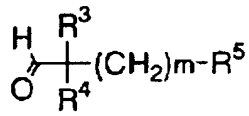

(IV)

and a heterocyclicmethylamine derivative represented by the following formula (V):

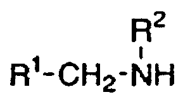

(V)

by using a reductive amination reaction,

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

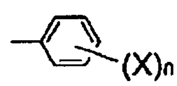

( II )

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

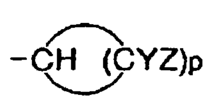

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different), when m is 1 and $R^4$ is a hydrogen atom, $R^5$ is a cycloalkyl group

described by the foregoing formula (III).]

**3.** A method of manufacturing a N-heterocyclicmethyl-alkylamine derivative including a process which obtains a N-heterocyclicmethyl-alkylamine derivative represented by the following formula (I):

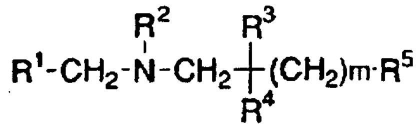

( I )

from an alkylamine derivative represented by the following formula (VI):

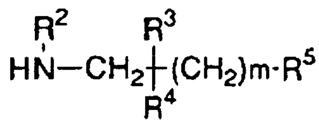

(VI)

and a heterocyclicmethylation agent represented by the following formula (VII):

$R^1$-$CH_2$·W (VII)

[in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

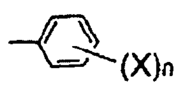

( II )

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and Beach represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

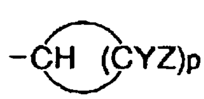

( III )

(in the formula, Y and Z may be the same or different from each other, and each represents one kind selected

from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different.), when m is 1 and $R^4$ is a hydrogen atom, $R^5$ is a cycloalkyl group described by the foregoing formula (III).]

4. The method of manufacturing the N-heterocyclicmethyl-alkylamine derivative according to claim 2, wherein the aldehyde derivative represented by the following formula (IV):

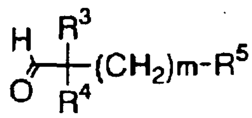

(IV)

which is obtained from the aldehyde derivative represented by the following formula (VIII):

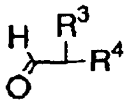

(VIII)

and the alkylation agent represented by the following formula (IX) :

$$W\text{-}(CH_2)m\cdot R^5 \qquad (IX)$$

is used,
[in the formula, $R^3$ represents one kind selected from a group consisting of an alkyl group having a carbon number of 1 to 5 and a halogenated alkyl group having a carbon number of 1 to 5; $R^4$ represents one kind selected from a group consisting of an alkyl group having a carbon number of 1 to 5 and a halogenated alkyl group having a carbon number of 1 to 5; carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

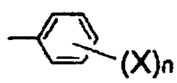

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

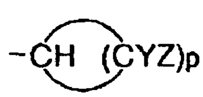

(III)

(in the formula, Y and Z may be the same or different from each other, and each represents one kind selected from a group consisting of a hydrogen atom, an alkyl group having a carbon number of 1 to 6, and a halogenated

alkyl group having a carbon number of 1 to 6, the letter p represents an integer of 2 to 5, and the groups described by CYZ may be each the same or different.); and W represents a leaving group.]

**5.** The method of manufacturing the N-heterocyclicmethyl-alkylamine derivative according to claim 2, wherein the aldehyde derivative represented by the following formula (IV):

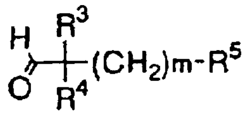

(IV)

which is obtained from the imine derivative represented by the following formula (X):

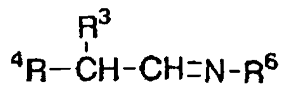

(X)

and the alkylation agent represented by the following formula ( IX) :

$$W\text{-}(CH_2)m\cdot R^5 \qquad (IX)$$

is used,
[in the formula, $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

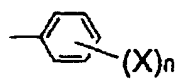

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

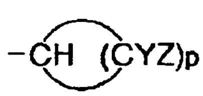

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described

by CYZ may be each the same or different) when m is 1 and $R^4$ is a hydrogen atom, $R^5$ is a cycloalkyl group described by the foregoing formula (III), $R^6$ represents one kind selected from a group consisting of an alkyl group having a carbon number of 2 to 6 and a cycloalkyl group having a carbon number of 3 to 6.]

**6.** The method of manufacturing the N-heterocyclicmethyl-alkylamine derivative according to claim 2, wherein the aldehyde derivative represented by the following formula (IV):

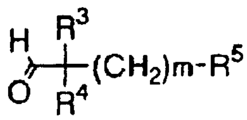

(IV)

which is obtained by reducing the ester derivative represented by the following formula (XI):

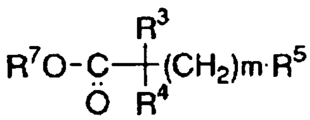

(XI)

is used,
[in the formula, $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

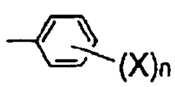

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6; a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and Beach represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

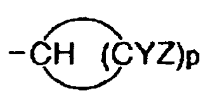

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different) , when m is 1 and $R^4$ is a hydrogen atom, $R^5$ is a cycloalkyl group described by the foregoing formula (III), $R^7$ represents an alkyl group having a carbon number of 1 to 3.]

**7.** The method of manufacturing the N-heterocyclicmethyl-alkylamine derivative according to claim 3, wherein the alkylamine derivative represented by the following formula (VI):

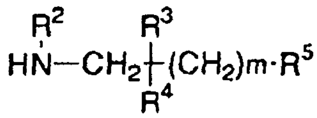

(VI)

which is obtained from the aldehyde derivative represented by the following formula (IV):

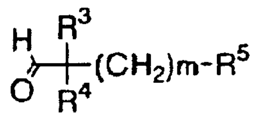

(IV)

and the amination agent represented by the following formula (XII) :

$$R^2\text{-}NH_2 \qquad \text{(XII)}$$

is used, by using a reductive amination reaction.
[in the formula, $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

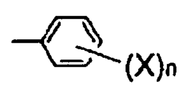

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

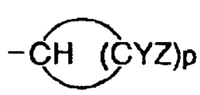

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different), when m is 1 and $R^4$ is a hydrogen atom, $R^5$ is a cycloalkyl group

described by the foregoing formula (III).]

**8.** The method of manufacturing the N-heterocyclicmethyl-alkylamine derivative according to claim 3, including a process which obtains the alkylamine derivative represented by the following formula (IV):

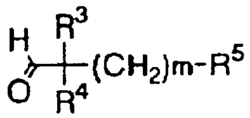

(IV)

by reducing the alkylamide derivative represented by the following formula (XIII):

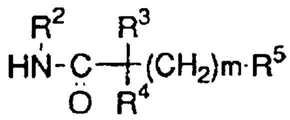

(XIII)

[in the formula, $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

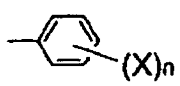

(II)

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6) , an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

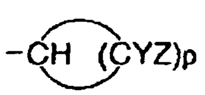

(III)

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different), when m is 1 and $R^4$ is a hydrogen atom, $R^5$ is a cycloalkyl group described by the foregoing formula (III).]

**9.** A fungicide containing a N-heterocyclicmethyl-alkylamine derivative represented by the following general formula

(I):

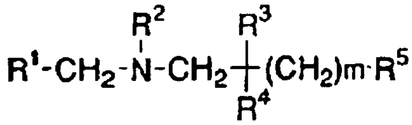

( I )

and an acid addition salt thereof as effective ingredients, [in the formula, $R^1$ represents a heterocycle which has at least one nitrogen atom as a hetero atom and may have a substituent on a ring; $R^2$ represents one kind selected from a group consisting of a hydrogen atom and an alkyl group having a carbon number of 1 to 5; $R^3$ represents one kind selected from a group consisting of an alkyl group having a carbon number of 1 to 5 and a halogenated alkyl group having a carbon number of 1 to 5; $R^4$ represents one kind selected from a group consisting of a hydrogen atom, an alkyl group having a carbon number of 1 to 5, and a halogenated alkyl group having a carbon number of 1 to 5; when $R^4$ is an alkyl group having a carbon number of 1 to 5 or a halogenated alkyl group having a carbon number of 1 to 5, carbon atoms in $R^3$ and $R^4$ may be bonded to form a ring structure; m represents an integer of 1 to 3; $R^5$ represents one kind selected from a group consisting of a phenyl group and a cycloalkyl group described by the following formulae (II) and (III), respectively, the formula (II) is:

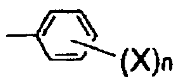

( II )

(in the formula, X represents one kind selected from a group consisting of a hydrogen atom, a halogen atom, an alkyl group having a carbon number of 1 to 6, a halogenated alkyl group having a carbon number of 1 to 6, an alkoxy group having a carbon number of 1 to 6, a halogenated alkoxy group having a carbon number of 1 to 6, a hydroxyalkyl group having a carbon number of 1 to 6, an alkoxyalkyl group (-AOB; A and B each represents an alkyl group having a carbon number of 1 to 6), a hydroxyiminoalkyl group having a carbon number of 1 to 6, an alkoxyiminoalkyl group (-A=N-OB; A and B each represents an alkyl group having a carbon number of 1 to 6), an acyl group, an ester group, a cyano group, a benzyl group optionally having a substituent on the ring, and a cycloalkyl group having a carbon number of 3 to 10; n represents an integer of 0 to 5; when n is 2 or more, X may be the same or different from each other and may be cross-linked to be condensed into a benzene ring forming a 5- or 6-membered ring.) and the formula (III) is:

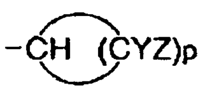

( III )

(in the formula, Y and Z may be the same or different from each other, and each represents one kind selected from a group consisting of a hydrogen atom, an alkyl group having a carbon number of 1 to 6, and a halogenated alkyl group having a carbon number of 1 to 6, the letter p represents an integer of 2 to 5, and the groups described by CYZ may be each the same or different) , when m is 1 and $R^4$ is a hydrogen atom, $R^5$ is a cycloalkyl group described by the foregoing formula (III).]

**INTERNATIONAL SEARCH REPORT**

International application No. PCT/JP01/03875

**A. CLASSIFICATION OF SUBJECT MATTER**

Int.Cl[7] C07D207/335, C07D213/38, C07D213/61, C07D241/12, C07D231/12, C07D239/26, C07D233/64 105, C07D277/32, C07D405/12, C07D401/12, C07D413/12, C07D249/02, A01N43/40 101, A01N43/16, A01N43/56, A01N43/54, A01N43/50, A01N43/36, A01N43/78, A01N43/653

According to International Patent Classification (IPC) or to both national classification and IPC

**B. FIELDS SEARCHED**

Minimum documentation searched (classification system followed by classification symbols)

Int.Cl[7] C07D207/335, C07D213/38, C07D213/61, C07D241/12, C07D231/12, C07D239/26, C07D233/64 105, C07D277/32, C07D405/12, C07D401/12, C07D413/12, C07D249/02, A01N43/40 101, A01N43/16, A01N43/56, A01N43/54, A01N43/50, A01N43/36, A01N43/78, A01N43/653

Documentation searched other than minimum documentation to the extent that such documents are included in the fields searched

Electronic data base consulted during the international search (name of data base and, where practicable, search terms used)

CA(STN), REGISTRY(STN), WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X | WO, 99/12902, A (Kureha Chemical Industry Co., Ltd.), 18 March, 1999 (18.03.99), | |
| | Claims 1,7 | 1,9 |
| | Claim 2 | 2 |
| | Claim 5 | 3 |
| | Claims 3, 5 | 7 |
| | Claims 4, 5 | 8 |
| A | entire description | 4-6 |
| | & EP, 1020441, A | |
| X | MASNER Petr et al., "Novel Inhibitors of Sterol C-14 Demethylase and $\Delta^{14}$ Reductase/$\Delta^{8} \rightarrow \Delta^{7}$ Isomerase for Cereal Disease Control", Pestic. Sci., Vol.35, No.4, pp.339-347(1992) | 1,3,7 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 11 June, 2001 (11.06.01) | 19 June, 2001 (19.06.01) |
| Name and mailing address of the ISA/ Japanese Patent Office | Authorized officer |
| Facsimile No. | Telephone No. |

Form PCT/ISA/210 (second sheet) (July 1992)